# EUROPEAN PATENT SPECIFICATION

(11) **EP 3 394 618 B1**
(45) Date of publication and mention of the grant of the patent: **23.10.2019**
(21) Application number: 16812943.5
(22) Date of filing: 15.12.2016
(51) Int. Cl.: G01N 33/558, G01N 33/68, G01N 33/58

(54) **DEVICE FOR DETECTING NEUROTOXINS AND PROCESS FOR MANUFACTURE THEREOF**
VORRICHTUNG FÜR DEN NACHWEIS VON NEUROTOXINEN UND VERFAHREN ZUR HERSTELLUNG DAVON
DISPOSITIF DE DÉTECTION DE NEUROTOXINES ET SON PROCÉDÉ DE FABRICATION

(30) Priority: 22.12.2015 WO PCT/EP2015/081060
(43) Date of publication of application: 31.10.2018
(73) Proprietor: Centre National de la Recherche Scientifique CNRS, 75794 Paris Cedex 16 (FR); Commissariat à l'Energie Atomique et aux Energies Alternatives (CEA), 75015 Paris (FR)
(72) Inventor: ARAOZ, Romulo, 91190 Gif-sur-Yvette (FR); MOLGO, Jordi, 92160 Antony (FR); SERVENT, Denis, 78000 Versailles (FR); MOURIER, Gilles, 91191 Gif-sur-Yvette Cedex (FR); KESSLER, Pascal, 91191 Gif-sur-Yvette Cedex (FR)
(74) Representative: Lavoix
(86) International application number: PCT/EP2016/081252
(87) International publication number: WO 2017/108582

(56) References cited:
- EP-A2- 1 376 124
- WO-A1-95/05601
- WO-A2-2008/073222
- US-A1- 2013 303 405
- TANG D ET AL: "Magnetic nanogold microspheres-based lateral-flow immunodipstick for rapid detection of aflatoxin B2 in food", BIOSENSORS AND BIOELECTRONICS, ELSEVIER BV, NL, vol. 25, no. 2, 15 October 2009 (2009-10-15), pages 514-518, XP026600460, ISSN: 0956-5663, DOI: 10.1016/J.BIOS.2009.07.030 [retrieved on 2009-08-04]

## Description

### Field of the Invention

The present invention relates to a device for detecting neurotoxins, a method for manufacturing an analysis device, and use of an analysis device detecting and quantifying neurotoxins.

The present invention also relates to a device for detecting a toxins and/or ligands targeting/for an ion-channel-linked receptor and/or a voltage gated ion-channel and use of an analysis device detecting and quantifying said ligand.

The present invention finds an application in the medical field and also in food field, in particular in the field of monitoring seafood, in the field of monitoring freshwater reservoirs, in the field of medical research, and in the field of the biological analysis and characterization of molecules.

In the description below, the reference between square brackets ([ ]) refer to the list of references given at the end of the text.

### Background of the Invention

Lateral flow tests are ready-to-use low-cost point-of-care diagnostic devices based on the affinity antigen-antibody, on enzymatic reactions, on DNA probes and on the use of aptamers (Chen and Yang (2015) Replacing antibodies with aptamers in lateral flow immunoassay. Biosensors & bioelectronics 71:230-242 [1]; Millipore (2013) Rapid lateral flow test strips: Considerations for product development. In: Lit No TB500EN00MM, Rev C: Merck Millipore [2]; Ngom et al. (2010) Development and application of lateral flow test strip technology for detection of infectious agents and chemical contaminants: A review. Analytical and Bioanalytical Chemistry 397:1113-1135 [3]). There are in the art of lateral flow test many devices/methods for detecting molecules in a sample, in particular in the medical field, for example for auto diagnose of pregnancy, self-monitoring of blood glucose, early diagnosis of infectious diseases, rapid identification of pathogen bacteria, detection of food contaminants, drugs, heavy metals and toxin detection (the list is not limitative) (Anfossi et al. (2013) Lateral-flow immunoassays for mycotoxins and phycotoxins: A review. Analytical and Bioanalytical Chemistry 405:467-480 [4]; Buhrer-Sekula et al. (2003) Simple and fast lateral flow test for classification of leprosy patients and identification of contacts with high risk of developing leprosy. Journal of Clinical Microbiology 41:1991-1995. [5]). Lateral flow tests are based on the formation of a complex between a detector particle which is free in the sample stream and a capture reagent that is bound to the membrane strip at the test line. The complex detector-target can be visualized by color precipitation, by fluorescence, chemiluminescence, electrochemical reactions (Marks et al. (2014) Electrochemical lateral flow bioassay and biosensor. WO2014171891 [6]).

TANG D ET AL: BIOSENSORS AND BIOELECTRONICS, ELSEVIER BV, NL, vol. 25, no. 2, 15 October 2009 (2009-10-15), pages 514-518 disclose a magnetic nanogold microspheres-based lateral-flow immunodipstick for rapid detection of aflatoxin B2 in food.

WO2008/073222 discloses a device for detecting an analyte in a liquid sample, wherein said device comprises porous material which provides for capillary flow of a liquid sample.

Most of lateral flow test are immunochromatography based, that is, using antibodies as detectors or targets. Herein is presented a lateral flow test based on the immobilization of biological membranes comprising neuro-receptors and/or ion channels for the detection of neurotoxins. NeuroTorp lateral flow test is based on the affinity TOXIN-RECEPTOR.

According to recent European directives, Directive 2010/63/EU [7], the "mouse test", which was the only official test used for monitoring marine toxins, must be replaced with prevalidated functional methods. Particularly in France, the "mouse test" was replaced with physicochemical methods for seafood health monitoring starting from 2010 (Closure of the Shellfish Farming Conference (10/15/2010). Speech by Bruno Le Maire, minister of food, agriculture and fisheries) (http://agriculture.gouv.fr/cloture-des-assises-de-la) [8].

Efflorescence of marine dinoflagellates, when they are dominated by toxic species form the so called Harmful Algal Blooms, representing a danger to the marine ecosystem, commercial activities and public health.

This is because the phycotoxins of dinoflagellates can be accumulated by mollusks and fish and, via vectorial transport, can reach humans (Fleming et al. (2006) Oceans and human health: Emerging public health risks in the marine environment. Marine Pollution Bulletin 53:545-560 [9], Molgó et al. (2007) Cyclic imines: an insight into this emerging group of bioactive marine toxins. In Phycotoxins: Chemistry and Biochemistry (Botana, L.M., ed) pp. 319-335, Blackwell Publishing Ltd, Iowa [10]). In 2005, REPHY, the French Phytoplankton Monitoring Network detected, by means of the "mouse test", mollusks contaminated with unknown fast-acting neurotoxins in samples from the Arcachon basin. Consequently, the French Government decreed that it was prohibited to consume oysters and shellfish from the Arcachon basin and had to expend 2.5 million Euros in aid for the shellfish industry of the Region of Arcachon, France. Later on, spirolide A and 13-desmethyl spirolide C, which are toxins produced by the dinoflagellate *Alexandrium ostenfeldii,* were detected in oysters and mussels from the Arcachon basin (Amzil et al. (2007) Report on the first detection of pectenotoxin-2, spirolide A and their derivatives in French shellfish. Marine Drugs 5:168-179 [11])

Cyclic imine toxins exhibit fast acting neurotoxicity and mouse lethality by respiratory arrest within minutes following intraperitoneal or oral administration. In the context of the ANR Neurospiroimine program (research program financed by the National Research Agency (ANR) (PCV07-1 9441 7-Neurospiroimine)), the mechanism of action of cyclic imine toxins of the spirolide and gymnodimine sub-family was established (Bourne et al. (2010) Structural determinants in phycotoxins and AChBP conferring high affinity binding and nicotinic AChR antagonism. Proceedings of the National Academy of Sciences of the United States of America, 107:6076-6081 [12]). Neurotoxins of the cyclic imine toxins family that comprises spirolides, gymnodimines, pinnatoxins, pteriatoxins, prorocentrolide and spiroprorocentrimine, are powerful antagonists of nicotinic acetylcholine receptors (nAChR) of muscle- and neuronal-type and possess affinities in the picomolar and nanomolar range (Bourne et al. (2010) [12], Kharrat et al. (2008) The marine phycotoxin gymnodimine targets muscular and neuronal nicotinic acetylcholine receptor subtypes with high affinity. Journal of Neurochemistry, 107, 952-963 [13], Araoz et al. (2011) Total synthesis of pinnatoxins A and G and revision of the mode of action of pinnatoxin A. Journal of the American Chemical Society, 133:10499-10511 [14]).

The current methods for detecting toxins in the shellfish industry, i.e. marine phycotoxins, are mainly:
- the "mouse test" which must be replaced with a test which does not use animals, by virtue of the change in European regulations. Currently in France, the "mouse test" is only used in the context of paralytic shellfish poisoning monitoring due to saxitoxin bioaccumulation ([8]). Furthermore, this test is difficult to carry out and requires considerable infrastructures (i.e. animal house) for its implementation. In addition, those working in shellfish farming question the validity of this test and contest the results obtained;
- High Performance Liquid Chromatography (HPLC). This method is expensive, requiring considerable equipment which is expensive and not very sensitive;
- HPLC coupled to mass spectrometry (LC-MS). This method is expensive, requiring considerable equipment which is expensive and qualified personnel in order to use said equipment. Despite its high sensitivity and specificity, this method requires toxin standards in order to calibrate the equipment for their detection. In addition, the LC-MS method has limited use in the context of the detection of unknown toxins. Since 2010, LC-MS has been the official method for detecting or monitoring marine phycotoxins which are subject to regulation in France, thus relegating the mouse test to the role of seafood paralytic shellfish poisoning monitoring ([8]). However, the LC-MS method does not make it possible to and cannot be used to detect novel toxins.
- Microplate receptor binding assay. The microplate-receptor binding assay is a target-directed functional method based on the mechanism of action of competitive agonists/antagonists of nAChRs for the detection of freshwater and marine neurotoxins such as cyanobacterial anatoxins or cyclic imine toxins (Araoz et al. (2012) Coupling the Torpedo microplate-receptor binding assay with mass spectrometry to detect cyclic imine neurotoxins. Analytical Chemistry 84:10445-53 [19]). The microplate-receptor binding assay is a high throughput method for rapid detection of freshwater and marine bioactive compounds targeting nAChRs directly in environmental samples such as cyanobacterial filaments, freshwater or shellfish extracts, with minimal sample handling, reduced matrix effect and toxin cross-reactivity, that could facilitate de use of mass spectrometry-based methods (Araoz et al. 2012 [19]). This method is difficult to apply directly in the field.

US2013303405 discloses a method for manufacturing an analysis substrate and its use for detecting toxins. A lateral flow format is not disclosed.

All these methods have to be carried out in laboratories and thus it implies a long time is needed to obtain a result whether there is a toxin or not in the sample. This is important because, in foodstuff, in particular in shellfish production, the sales have to be stopped during the uncertainty time (is there any toxin in the growing environment of shellfish?) and could lead to destruction of food and/or legal advertise to the consumers to stop eating shellfish in order to avoid public health problems.

There is therefore a real need to find a method and/or a device which is simple and inexpensive, for example a lateral flow test for detecting toxins, in particular neurotoxins, for example for monitoring neurotoxic phytoplankton for the shellfish industry or the quality of bathing water along tourist beaches, and also for monitoring contaminated seafood.

In particular, there is a need to find a device/method (i.e. lateral flow test) which allows to determine directly and in a short time whether there is a toxin in a sample.

Similarly, the proliferation of cyanobacteria in freshwater reservoirs constitutes a potential danger to public health. This is because cyanobacterial species can produce neurotoxins; they may, for example, be toxic species of the *Anabaena* or *Oscillatoria* genus or others producing anatoxin-a or homoanatoxin-a (Sivonen et al. (1999) Cyanobacterial toxins. In Toxic cyanobacteria in water: a guide to their public health consequences, monitoring and management (Chorus I., ed) pp. 41-111, Bartram, J.E. & F.N. Spon, London [16]). Admittedly, numerous episodes of poisoning of dogs having drunk water with a high content of cyanobacteria producing anatoxin-a and homoanatoxin-a, which are powerful nAChR agonists, have occurred in France in rivers such as the Loue (2003) and the Tarn (2002, 2003 and 2005) (Gugger et al. (2005) First report in a river in France of the benthic cyanobacterium Phormidium favosum producing anatoxin-a associated with dog neurotoxicosis. Toxicon 45:919-928 [17], Cadel-Six et al. (2007) Different genotypes of anatoxin-producing cyanobacteria coexist in the Tarn River, France. Applied and Environmental Microbiology 73:7605-7614. [18]).

The current methods for detecting cyanobacterial neurotoxins are mainly:
- HPLC: this method is expensive, requiring considerable equipment which is expensive and not very sensitive.
- HPLC coupled to mass spectrometry (LC-MS). This method is expensive and requires considerable equipment which is expensive, and also qualified personnel in order to use this equipment.
- Microplate receptor binding assay. The microplate-receptor binding assay is a target-directed functional method based on the mechanism of action of competitive agonists/antagonists of nAChRs for the detection of freshwater and marine neurotoxins such as cyanobacterial anatoxins or cyclic imine toxins (Aráoz et al. 2012 [19]). Microplate receptor binding assay is nowadays commercialized by ABRAXIS for cyclic imine toxins detection, and the conditions were optimized for anatoxin-a detection (Rubio et al. (2014) Colorimetric microtiter plate receptor-binding assay for the detection of freshwater and marine neurotoxins targeting the nicotinic acetylcholine receptors. Toxicon 91:45-56 [20]).

There are also in the prior art methods for the detection in solution of compounds such as 13,19-didesmethyl spirolide C, gymnodimine A and 13-desmethyl spirolide C (Fonfria et al. (2010) Detection of 13,19-didesmethyl spirolide C by fluorescence polarization using Torpedo electrocyte membranes. Analytical Biochemistry, 403:102-107 [21], Vilariño et al. (2009) Detection of gymnodimine-A and 13-desmethyl spirolide C phycotoxins by fluorescence polarization. Analytical Chemistry 81:2708-2714 [22]) or of cyanobacterial anatoxin-a and homoanatoxin-a (Araoz et al. (2008) A non radioactive ligand-binding assay for detection of cyanobacterial anatoxins using Torpedo electrocyte membranes. Toxicon, 52:163-174 [23]). However, these methods have low detection sensitivities and require for the detection of said compounds the use of considerable equipment, such as polarization fluorimeters, and chemiluminescence detectors which are expensive and complex, thus requiring qualified personnel for their implementation and use.

There is therefore a real need to find a method and/or a device (i.e. lateral flow test) which is simple and inexpensive, for example a functional test for detecting toxins, in particular neurotoxins, for example for monitoring neurotoxic cyanobacteria and the quality of freshwater reservoirs for the drinking-water processing industry, or the quality of freshwater bodies (lakes, ponds, rivers) used for recreational activities, (canoeing, fishing, aquatic sports) or as drinking water for pets and stock animals, and also for monitoring contaminated fish.

In particular, there is a need to find a lateral flow test device/method which allows to determine directly and in a short time whether there is a toxin in a sample.

In addition, there is a need to find a method/device that allow to detect directly and in a short delay whether there is a toxin in a sample, in particular to detect if there is a toxin whatever is the toxin, for example saxitoxins, paralyzing toxins, cyanobacterial neurotoxins and/or marine phycotoxins.

### Description of the invention

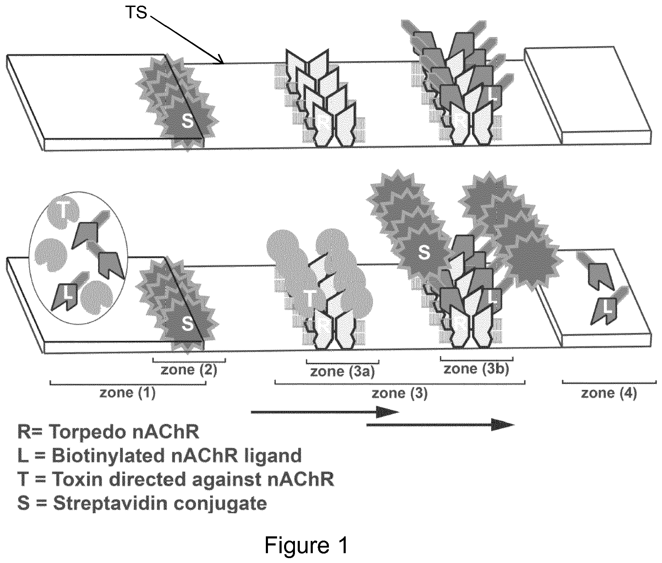

The present invention allows to solve and to overcome the abovementioned obstacles and drawbacks of the prior art by providing an *in-vitro* device for detecting toxins, in particular neurotoxins, for example as disclosed in Figure 1, comprising:
- a zone (1) for depositing a sample;
- a zone (2) comprising a conjugate comprising an enzyme coupled to a molecule which bind to a tagged ligand of a ion-channel-linked receptor and/or a voltage-gated ion-channel or which bind to an antibody directed against the neurotoxin binding site of the ion-channel-linked receptor and/or a voltage gated ion-channel, and/or comprising a nanogold coated molecule, a carbon-black coated molecule or a fluorescent molecule that binds to a tagged ligand of an ion-channel-linked receptor and/or a voltage gated ion-channel
- a visualizing zone (3) comprising
   ∘ a test zone (3a) comprising fragmented and isolated cell membranes comprising an ion-channel-linked receptor and/or a voltage gated ion-channel fixed on a test surface, and
   ∘ a control zone (3b) comprising fragmented and isolated cell membranes comprising ion-channel-linked receptor and/or a voltage gated ion-channel associated with a tagged ligand of the ion-channel-linked receptor and/or a voltage gated ion-channel, said ligand being fixed on a test surface and /or a control zone (3b') comprising a tagged ligand of the ion-channel-linked receptor and/or a voltage gated ion-channel, said ligand being fixed directly on a test control surface with the technical features defined in the corresponding claims.

In other words, the present invention also provides an *in-vitro* device for detecting in a sample neurotoxins or an ion-channel-linked receptor, and/or a voltage gated ion-channel ligand comprising:
- a zone (1) for depositing a sample;
- a zone (2) comprising
   ∘ a conjugate comprising an enzyme coupled to a molecule which binds to a tagged ligand of an ion-channel-linked receptor, and/or a voltage gated ion-channel, or
   ∘ a conjugate comprising an enzyme coupled to a molecule which binds to an antibody directed against the neurotoxin binding site of the ion-channel-linked receptor and/or of the voltage gated ion-channel, and/or
   ∘ a conjugate comprising a nanogold coated molecule which binds to a tagged ligand of an ion-channel-linked receptor, and/or a voltage gated ion-channel, or
   ∘ a conjugate comprising a carbon-black coated molecule which binds to a tagged ligand of an ion-channel-linked receptor, and/or a voltage gated ion-channel, or
   ∘ a conjugate comprising a fluorescent molecule that binds to a tagged ligand of an ion-channel-linked receptor and/or a voltage gated ion-channel
- a visualizing zone (3) comprising
   ∘ a test zone (3a) comprising fragmented and isolated cell membranes comprising an ion-channel-linked receptor and/or a voltage gated ion-channel fixed on the test surface, and
   ∘ a control zone (3b) comprising fragmented and isolated cell membranes comprising an ion-channel-linked receptor and/or a voltage gated ion-channel bound with a tagged ligand of the ion-channel-linked receptor and/or of the voltage gated ion-channel, said ion-channel-linked receptor and/or a voltage gated ion-channel being fixed on the test surface or
   ∘ a control zone (3b') comprising a tagged ligand of the ion-channel-linked receptor and/or of the voltage gated ion-channel, said ligand being fixed directly on the test surface
   and
- an absorption zone (4) as defined in claim 1.

In the present by "associated" or "bound" means for example, a molecular interaction such as hydrogen, ionic, or van der Waals bonding interactions, a biological interaction, for example, specific three-dimensional hormone-receptor pattern recognition or antibody-antigen interactions, an electrostatic interaction, a concentration gradient of ions or molecules, in other words, any structural features within the ion-channel-linked receptor binding site or the voltage gated ion channel binding-site, and the structural features within the toxin and/or the ligand that drive the interaction and the binding of the toxin/ ligand to the binding site of the ion-channel-linked receptor and/or of the voltage gated ion-channel with high affinity, for example via hydrogen bonds, van der Waals interactions, hydrophobic bonds, covalent bonds, ionic bonds.

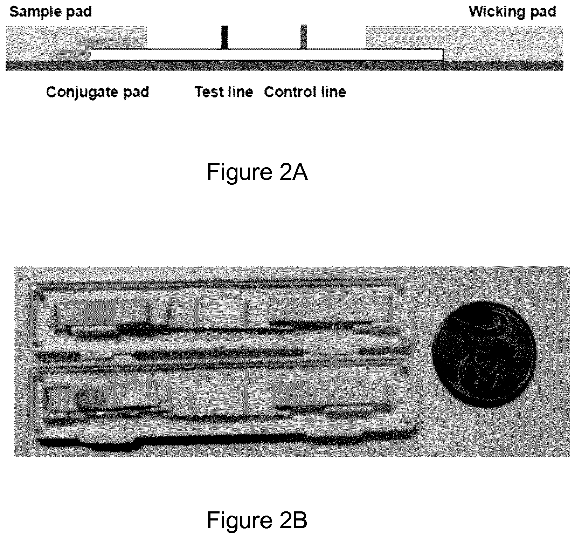

In the present, the *in-vitro* device for detecting toxins of the present invention is also referred to as *in-vitro* lateral flow test device. For example, it may be the device represented in Figure 2A.

In the present invention, the sample may be any sample, for example environmental samples, known from one skilled in the art that could contain toxin. It may be for example a sample of water, for example from a sewage treatment industry, a sample of sea water, a sample of water from a shellfish farming industry, a sample of water from freshwater reservoirs, a sample of drinking water. It may be also a biological sample, for example a sample of any biological fluid, for example of blood, of milk, of urine, a sample of biological tissue obtained from a biopsy. The sample could be an extract of shellfish, for example from oysters, clams, mussels, an extract of phytoplankton, for example from dinoflagellates, diatoms, an extract of cyanobacteria, an extract of bacteria, an extract of plants, an extract of vertebrate and invertebrate animals, for example a fish extract.

In the present invention, the sample may be any sample, for example environmental samples, known from one skilled in the art that could contain toxin, neurotoxin and/or a ligand of an ion-channel-linked receptor and/or a voltage gated ion-channel. It may be for example a sample of water, for example from a sewage treatment industry, a sample of sea water, a sample of water from a shellfish farming industry, a sample of water from freshwater reservoirs, a sample of drinking water. It may be also a biological sample, for example a sample of any biological fluid, for example of blood, of milk, of urine, a sample of biological tissue obtained from a biopsy. The sample could be an extract of shellfish, for example from oysters, clams, mussels, an extract of phytoplankton, for example from dinoflagellates, diatoms, an extract of cyanobacteria, an extract of bacteria, an extract of plants, an extract of vertebrate and invertebrate animals, for example a fish extract.

In the present "extract" means any part/sample that could be obtained from shellfish, a vertebrate and/or an invertebrate animal, from plants, from higher plants, form marine plants, from macroalgae, from microalgae, from dinoflagellates, from cyanobacteria, from bacteria.

In the present invention, the toxin may be any toxin known by one skilled in the art and that could bind and/or be fixed to an ion-channel-linked receptor, for example a ligand gated ion-channel-linked receptor and/or a voltage gated ion-channel. It may be, for example, a toxin selected from the group comprising neurotoxins produced by animals, for example from snakes and/or frogs, plants, mollusks, microorganisms, for example from dinoflagellates, diatoms, cyanobacteria. It may be for example fast acting toxins, for example anatoxin-a for example from toxic fresh-water cyanobacteria, or pinnatoxin-A for example from toxic dinoflagellates, both acting on nAChRs, or saxitoxin for example from freshwater cyanobacteria or marine dinoflagellates acting on voltage gated sodium channels. These toxins for example anatoxin-a, spirolides, saxitoxins, are "fast acting" toxins that are known to induce rapid onset of neurotoxic symptoms and death within few minutes following intraperitoneal administration in mice.

According to the invention, the neurotoxins may be any neurotoxins known to those skilled in the art; for example, they may be neurotoxins which act, for example, on nicotinic acetylcholine receptors, neurotoxins produced by marine phytoplankton, such as certain members of the *Alexandrium* genus, for example *Alexandrium ostenfeldii,* producing, for example, spirolide, members of the *Karenia* genus, for example *Karenia selliformis,* producing, for example, gymnodimine, phycotoxins of the cyclic imine toxin family, for example pinnatoxins for example produced by *Vulcanodinium rugosum,* pteriatoxins, prorocentrolides or spiro-prorocentrimine, which may be produced by various phytoplankton species (Molgó et al. 2007 [10]). They may also be cyanobacterial neurotoxins, for example anatoxin-a or homoanatoxin-a, produced, for example, by members of the *Anabaena, Aphanizomenon, Cylindrospermum,* Microcystis, *Oscillatoria, Phormidium, Planktothrix* and *Raphidiopsis* genera, or pinnamine, a marine toxin with a chemical structure very close to anatoxin-a, and/or any toxin capable of acting on nAChRs (Aráoz et al. (2009) Neurotoxic cyanobacterial toxins. Toxicon 56:813-828 [23], Sivonen et al. 1999 [16].

In the present invention, the ion-channel-linked receptor or a voltage gated ion-channel may be any ion-channel-linked receptor or a voltage gated ion-channel known from one skilled in the art. It may be an ion-channel-linked receptor selected from the group comprising ligand gated channels receptor, for example nicotinic acetylcholine receptor, glycine receptor and/or NMDA receptor, or voltage-gated channel receptor, for example voltage gated sodium channel, voltage-gated potassium channel. Preferably the ion-channel-linked receptor is the nicotinic acetylcholine receptor.

In the present invention the ligand of the ion-channel-linked receptor may be any ligand of the ion-channel-linked receptor known in the art. For example when the ion-channel-linked receptor is nicotinic acetylcholine receptor the ligand may be selected from the group comprising snake peptides, for example: α-bungarotoxin, conus peptides, for example α-conotoxines. For example when the ion-channel-linked receptor is a voltage-gated sodium channel the ligand may be selected from the group comprising venom peptides, for example µ-conotoxin (KIIIA) and/or δ-conotoxins. For example when the ion-channel-linked receptor is voltage-gated potassium channel the ligand may be selected from the group comprising spider and/or scorpion toxins, for example spider, and/or scorpion peptide toxins.

In the present invention, the ligand of the ion-channel-linked receptor may be for example an antibody directed against the neurotoxin binding site within the receptor ion channel.

One in the art taking into consideration his knowledge and the state of the art would be able to select the ligand with regards to the ion-channel-linked receptor or a voltage gated ion-channel.

In the present the ligand of an ion-channel-linked receptor and/or a voltage gated ion-channel may be tagged with any tag adapted and known from one in the art. It may be for example a tag selected from the group comprising biotin, fluorescent dyes for example rhodopsine, alexa-Fluor, nanogold coated ligands, carbon-black coated ligands, or a fluorescent ligand. In the present a conjugate molecule which bind to a tagged ligand may be any molecule know from one in the art to bind a tagged ligand. For example, when the tag is biotin the conjugate molecule may be streptavidine.

In the present invention the enzyme coupled to molecule which binds to a tagged ligand of an ion-channel-linked receptor may be any adapted enzyme known from one in the art. It may be for example an enzyme selected from the group comprising the enzyme peroxidase, betagalactosidase, glucose oxidase and alkaline phosphatase. Preferably, the enzyme is alkaline phosphatase, it may be for example an alkaline phosphatase available in the market, for example an alkaline phosphatase sold by Promega or Sigma.

According to the invention, when using an enzyme, the substrate of the enzyme may be any substrate known from one in the art. It may be, for example a substrate selected from the group comprising 3,3',5,5'-tetramethylbenzidine (also called Membrane TMB), 4-chloro-1-naphtho / 3,3'-diaminobenzidine tetrahydrochloride (also called CN/DAB), 3-amino-9-ethylcarbazole (also called AEC), 3,3-dimethoxybenzidine-o-dianisidine (also called ODN), 5-bromo-4-chloro-3'-indolyl phosphate p-toluidine salt (also called BCIP), a mixture of nitro-blue tetrazolium chloride (also called NBT) and of 5-bromo-4-chloro-3'-indolyl phosphate p-toluidine salt (also called BCIP), the mixture Naphthol As-Mx phosphate and 4-chloro-2-methylbenzenediazonium salt (also called Fast red TR salt), the mixture Naphthol As-Mx phosphate and diazotized salt of 4'-amino-2',5'-diethoxybenzanilide zinc chloride (also called Fast blue BB salt), 5-iodo-3-indolyl-p-D-galactopyranoside (also called Purple-Gal), 5-bromo-6-chloro-3-indolyl-β-D-galactopyranoside (also called Red-Gal), 6-chloro-3-indolyl-β-D-galactopyranoside (also called Rose-Gal), 5-bromo-3-indolyl-β-D-galactopyranoside (also called Blue-Gal), N-methylindolyl-β-D-galactopyranoside (also called Green-Gal). One in the art taking into consideration his knowledge and the state of the art would be able to select the substrate with regards to the enzyme coupled to a molecule which binds to a tagged ligand of an ion-channel-linked receptor.

In the present a molecule that binds to a tagged ligand of an ion-channel-linked receptor and/or a voltage gated ion-channel may be a nanogold coated molecule, a carbon-black coated molecule or a fluorescent ligand.

In another example, when the ligand is tagged with a fluorescent dye there is no need of a conjugate molecule. The detection of the complex ligand-receptor is made directly by fluorescence. In the same manner, when the ligand is labelled with colloidal gold, the detection of the complex ligand-receptor is made directly by the naked eye.

In the present the fragmented and isolated cell membranes comprising an ion-channel-linked receptor and/or voltage gated ion channels may be obtained from any cell know from one skilled in the art. It may be for example fragmented and isolated membranes obtained from electrocyte cell membrane, native mammalian neuronal cells, mammalian neuronal cells genetically modified. It may be for example fragmented and isolated membranes obtained from isolated electrocyte cells, from mammalian neuronal cells, for example mammalian neuronal cells in culture, from immortalized human cells in culture, for example transfected with a given ligand gated receptor channel or voltage gated ion channel, for example HEK from Human Embryonic Kidney cells, and/or from mammalian immortalized cell lines (CHO from Chinese Hamster Ovarian cells). It may be for example fragmented and isolated cell membranes from electrocyte cells of electric fish, for example of the species of the Torpedinidae family, such as Torpedoes, or of the family Electrophoridae, such as the electric eel (*Electrophorus electricus*)*.* It may be for example fragmented and isolated cell membranes from the cells that may be cells derived from the Torpedo electric organ, for example, electrocytes of electric ray, for example selected from the family of Torpedinidae, for example selected from the group comprising *Torpedo adenensis, Torpedo alexandrinsis* or *Alexandrine Torpedo, Torpedo andersoni* or *Florida Torpedo, Torpedo bauchotae, Torpedo californica* or Pacific electric ray, *Torpedo fairchildi, Torpedo fuscomaculata, Torpedo mackayana, Torpedo macneilli, Torpedo marmorata,* or marbled electric ray or marbled Torpedo ray, *Torpedo microdiscus, Torpedo nobiliana* or Atlantic electric ray, *Torpedo panthera* or Panther Torpedo ray, *Torpedo peruana,* Torpedo semipelagica, *Torpedo sinuspersici, Torpedo suessii, Torpedo tokionis, Torpedo Torpedo* or common Torpedo, and *Torpedo tremens.* Preferably, the cells used are cells of *Torpedo marmorata* or *Torpedo californica.*

It may be for example fragmented and isolated cell membranes from any mammalian neuronal cell known from one skilled in the art. It may be, for example neuronal cells obtained from a biopsy, neuronal cells lines, for example neuronal cells genetically modified, for example with an expression vector, expressing ion-channel-linked receptor. Fragmented and isolated cell membranes, for example from electrocyte cells, may be obtained according to the method and process described in PCT publication WO2012/101378.

In the present, the test surface may be a support of any material known to those skilled in the art, for example a material chosen from the group comprising absorbent paper, cotton, cellulose, glass fiber, and a nitrocellulose membrane. It may be for example a filter membrane manufactured with borosilicate glass fibers. It may be for example a glass fiber support commercially available, for example Whatman (registered trademark) glass microfiber filters, binder free, grade GF/ C GF/ A, GF/ B commercialized by Sigma. It may be for example nylon or polycarbonate membranes of high porosity, for example comprising pores that have a diameter from 1 to 8 µm, for example with 1, 3, 5 or 8 µm pore size. It may be for example nitrocellulose membranes of high porosity, for example comprising pores that have a diameter from 1 to 8 µm, for example with 1, 3, 5 or 8 µm pore size. It may be for example nitrocellulose membranes of high porosity commercialized by Sartorious. It may be for example mixed cellulose ester membranes, cellulose acetate membranes, hydrophilic PTFE membranes, Nylon or Polycarbonate membranes commercialized by Advantec. Preferably, the test surface is a glass fiber support, in particular borosilicate glass microfiber filters.

In the present the test surface may be a flat support or support with a particular design, for example rectangular, circular

The test surface may be for example a fiber support, for example comprising pores with a diameter from 0.8 µm to 8 µm, for example from 1 µm to 1,6 µm.

In the present the test surface may have a thickness from 0.08 mm to 0.350 mm, for example from 0.100 to 0.500 mm, for example equal to 0.26 mm.

Advantageously, the inventors have unexpectedly demonstrated that when the test surface is a fiber support with a pore diameter from 1 µm to 6 µm: *i*) it advantageously allows to fix fragmented and isolated cell membranes onto its surface, and ii) it advantageously allows to concentrate the torpedo-receptors in a narrow band. The inventors have also surprisingly demonstrated that when the fragmented and isolated cell membranes are fixed on a glass fiber support it allows the proteins, in particular the ion-channel-linked receptors present in the membrane fragments, to fully keep their biological activity regarding the binding properties of the receptors with any of their ligands.

According to the invention, zone (1) also designated herein the depositing zone (1) may be any zone suitable for the application or the reception of a sample. This zone may be of any form known to those skilled in the art, for example a reservoir, a cupule, a well, a wick or a flat surface.

According to the invention, the depositing zone (1) may be mobile and/or linked to the zone (2) or (3). When the zone (1) is mobile, it may be, for example, used to take the sample and applied to the zone (2). When the zone (1) is linked to the zone (2) or (3), it may be immersed directly in a container comprising the sample and/or the sample may be applied to this zone.

Advantageously, according to the invention, the zones (1) and (2) are located on the same support.

According to the invention, the materials/test surface of the zones (1) to (3) may be identical or different. According to the present invention, the materials/test surface is as defined above. Preferably, the depositing zone (1) may be, for example, a glass fiber support, a filter membrane manufactured with borosilicate glass fibers or a nylon or polycarbonate membrane of high porosity as mentioned above. More preferably the material zones (1) to (3) are a glass fiber support.

The inventors have surprisingly demonstrated that the use of a glass fiber supports allows surprisingly to fix and concentrate fragmented and isolated cell membranes comprising an ion-channel-linked receptor onto its surface without any degradation of the receptor activity nor any degradation of the structure of fragmented and isolated cell membranes comprising the ion-channel-linked receptor. In other words, the inventors have surprisingly demonstrated that the receptor fixed on the glass fiber support keep all its biological properties; in particular, its binding site is still effective.

In the present, the conjugate zone (2) may comprise a quantity of enzyme coupled to a molecule which bind to a tagged ligand of an ion-channel-linked receptor and/or a voltage gated ion-channel, or nanogold coated macromolecule which bind to a tagged ligand of an ion-channel-linked receptor and/or a voltage gated ion-channel, or carbon black coated macromolecule which bind to a tagged ligand of an ion-channel-linked receptor and/or a voltage gated ion-channel or an antibody directed against the neurotoxin binding site of the ion-channel-linked receptor. One in the art taking into consideration his knowledge would be able to select the volume of solution comprising enzyme coupled to a molecule to be applied to the conjugate zone (2) to obtain the corresponding protein quantity to visualize the complex receptor-ligand.

In the present, the test zone (3a) may comprise a quantity of fragmented and isolated cell membranes, for example Torpedo electric cells membranes, with a total protein concentration ranging from 10 to 500 µg/mL. In other words, the fragmented and isolated cell membranes comprising ion-channel-linked receptor and/or a voltage gated ion-channel in the test zone (3a) may be fixed and concentrated onto the narrow test zone (3a) from a stock solution comprising fragmented and isolated cell membranes in a concentration ranging from 10 to 500 µg/mL total protein. One skilled in the art taking into consideration his knowledge would be able to select the volume of solution comprising fragmented and isolated cell membranes comprising ion-channel-linked receptor and/or a voltage gated ion-channel to be applied to the test zone (3a) to obtain the corresponding protein quantity.

In the present, the test control zone (3b) may comprise a quantity of fragmented and isolated cell membranes comprising ion-channel-linked receptor and/or a voltage gated ion-channel associated with a tagged ligand from 10 to 500 µg/mL of total protein. In other words, the fragmented and isolated cell membranes comprising ion-channel-linked receptor and/or a voltage gated ion-channel associated with a tagged ligand in the control zone (3b) may be fixed and concentrated in the narrow control zone (3b) from a stock solution comprising fragmented and isolated cell membranes in a concentration from 10 to 500 µg/mL total protein. One in the art taking into consideration his knowledge would be able to select the volume of solution comprising fragmented and isolated cell membranes comprising ion-channel-linked receptor and/or a voltage gated ion-channel associated with a tagged ligand to be applied to the test zone (3a) to obtain the corresponding protein quantity.

In the present, the control zone (3b') may comprise a quantity of a tagged ligand of the ion-channel-linked receptor and/or a voltage gated ion-channel from 10 to 500 µg/mL. The tagged ligand of the ion-channel-linked receptor and/or a voltage gated ion-channel in the control zone 3b' may be a tagged ligand of the ion-channel-linked receptor and/or a voltage gated ion-channel as defined above. It may be for example a biotinylated molecule to which streptavidine, nanogold or carbon-black coated conjugate may bind.

According to the invention, the abovementioned various zones (1) to (3) can be attached to a solid support, for example to laminated cards, and/or included in a container comprising a window at the level of the zone (3) allowing the visualization of the result and a well at the level of the zone (1) for depositing the sample, allowing the deposition of the sample.

According to the invention, the device may also comprise an absorption zone (4), for example as disclosed in Figure 1. According to the invention, the absorption zone (4) may be any absorbent solid support known to those skilled in the art. It may, for example, be an absorbent blotting paper. It may, for example, be also an absorbent blotting paper, an absorbent filter paper and/or mixture thereof.

According to the invention the absorption zone (4), for example comprising an absorbent filter paper, may be able to drive the movement of the deposited sample also called mobile phase for example by capillarity.

In the lateral flow test device of the present invention, the supports of zones (1) and (2) may overlap at one of their ends, and the other end of zone (2) may overlap with one end of zone (3). The overlapping of the various zones (1) to (3) advantageously makes it possible, when the sample is applied and/or when the free end of zone (1) is immersed in the sample, for the sample to migrate in the various zones via capillary action. Preferably, zones (1), (2) and (3) are arranged on the same test surface For example Figure 2 represents an example of the lateral flow test device of the present invention.

Preferably, absorption zone (4) overlaps with the free end of the zone (3). Thus, zone (4) allows accelerated migration of the specimen through the various zones of the device by capillarity. Advantageously, the zone (4) makes it possible to absorb the excess liquid of the sample.

According to the invention, the various zones (1) to (4) may be independently covered with a protective film. This may, for example, be a plastic film, for example a polyvinyl chloride (PVC) film, or a biodegradable film, for example a polycaprolactone (PCL), polyvinyl alcohol (PVA) or polylactic acid (PLA) film.

Advantageously the film independently protects the various zones of the device of the invention.

Another objet of the present invention is a method of manufacturing an analysis device as defined in claims 1-8 comprising membrane fragments immobilized and /or fixed at the surface thereof, comprising the steps of:
a. attaching and concentrating fragmented and isolated cell membranes to the test surface of the device by filtration through the test surface of a first solution comprising said fragmented and isolated cell membranes,
b. attaching an ion-channel-linked receptor and/or a voltage gated ion-channel associated with a tagged ligand of the ion-channel-linked receptor and/or of the voltage gated ion-channel, said ligand being fixed and concentrated to the test surface of the device by filtration through the test surface of a second solution comprising said ion-channel-linked receptor and/or a voltage gated ion-channel associated with a tagged ligand of the ion-channel receptor and/or a voltage gated ion-channel, or attaching a tagged ligand of the ion-channel-linked receptor and/or a voltage gated ion-channel, said ligand being fixed directly on a test control surface.
c. attaching a conjugate comprising an enzyme coupled to a molecule which bind to a tagged ligand of an ion-channel-linked receptor and/or a voltage-gated ion-channel or which bind to an antibody directed against the neurotoxin binding site of the ion-channel-linked receptor and/or a voltage gated ion-channel, and/or comprising a nanogold coated molecule, a carbon-black coated molecule or a fluorescent molecule that binds to a tagged ligand of a ion-channel-linked receptor and/or a voltage gated ion-channel by immersion of the test surface in a third solution comprising said conjugate,
d. drying the test surface,
e. assembling and attaching the test surface onto a solid support.

In other words, another object of the present invention is method for manufacturing an analysis device as defined in claims 1-8 comprising membrane fragments immobilized at the surface thereof, comprising the steps of:
a. Attaching and concentrating fragmented and isolated cell membranes to the test surface of the device by filtration through the test surface of a first solution comprising said fragmented and isolated cell membranes,
b. Attaching and concentrating an ion-channel-linked receptor and/or a voltage gated ion-channel bound with a tagged ligand of the ion-channel-linked receptor and/or of the voltage gated ion-channel, said ion-channel-linked receptor and/or a voltage gated ion-channel being fixed to the control test surface of the device by filtration through the test surface of a second solution comprising said ion-channel-linked receptor and/or voltage gated ion-channel bound with a tagged ligand of the ion-channel-linked receptor and/or of the voltage gated ion-channel, or
   attaching a tagged ligand of the ion-channel-linked receptor and/or of the voltage gated ion-channel, said ligand being fixed directly on a test control surface.
c. attaching a conjugate comprising an enzyme coupled to a molecule which bind to a tagged ligand of an ion-channel-linked receptor and/or to a voltage-gated ion-channel or a conjugate which bind to an antibody directed against the neurotoxin binding site of the ion-channel-linked receptor and/or of the voltage gated ion-channel, and/or a conjugate comprising a nanogold coated molecule, a carbon-black coated molecule or a fluorescent molecule that binds to a tagged ligand of an ion-channel-linked receptor and/or of a voltage gated ion-channel by immersion of the test surface in a third solution comprising said conjugate.
d. drying the test surface,
e. assembling and attaching the test surface onto a solid support.

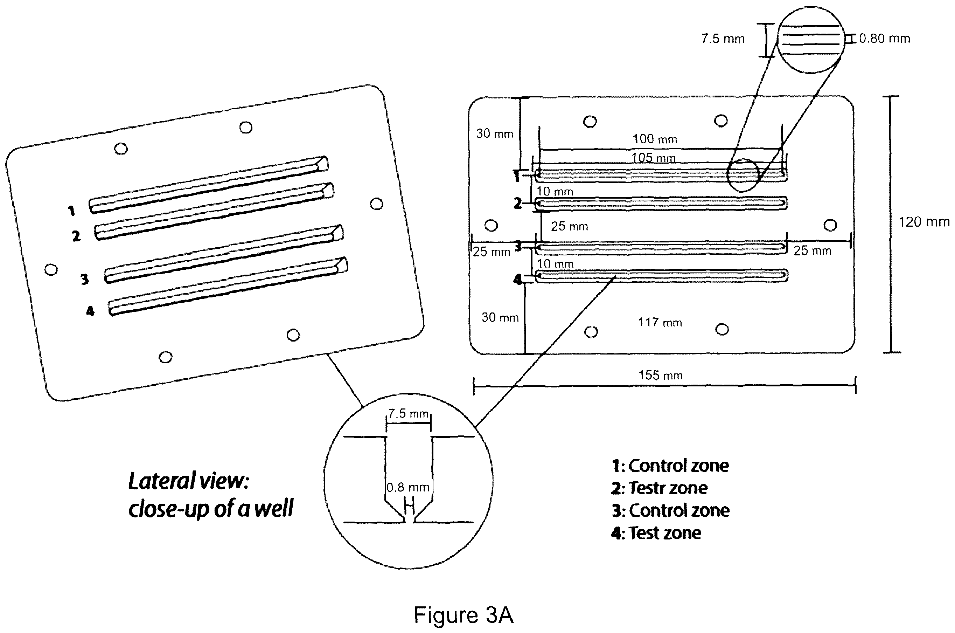

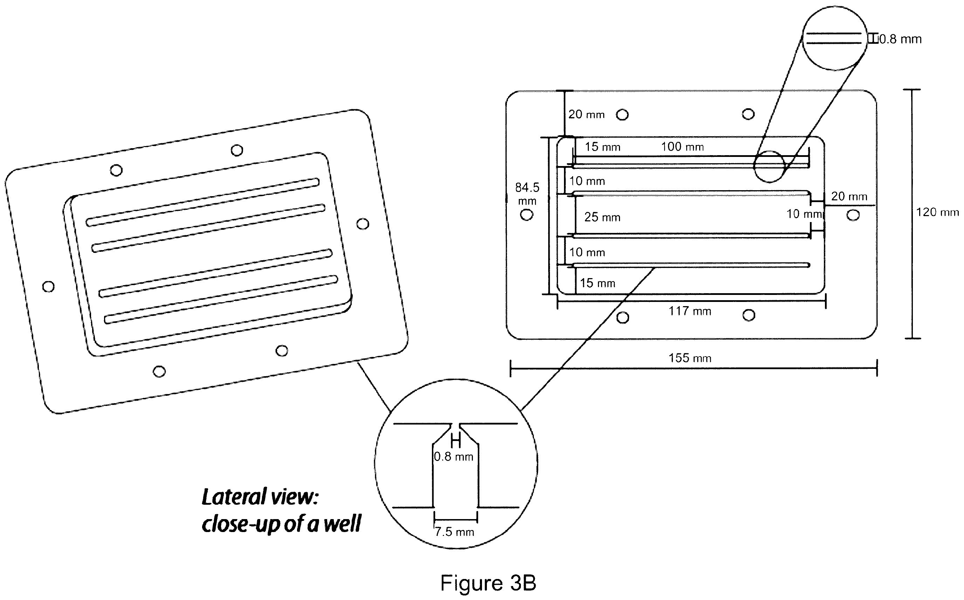

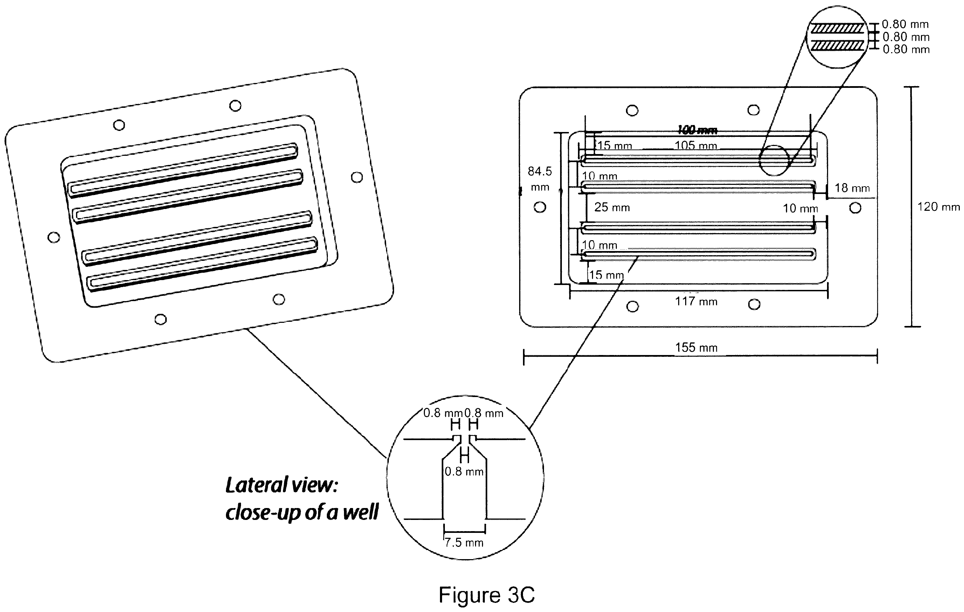

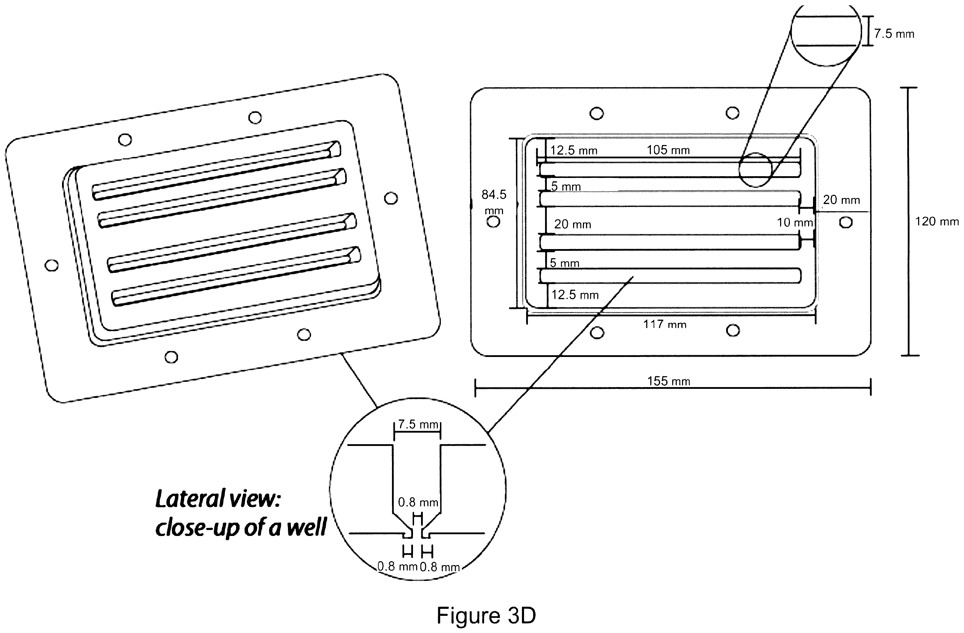

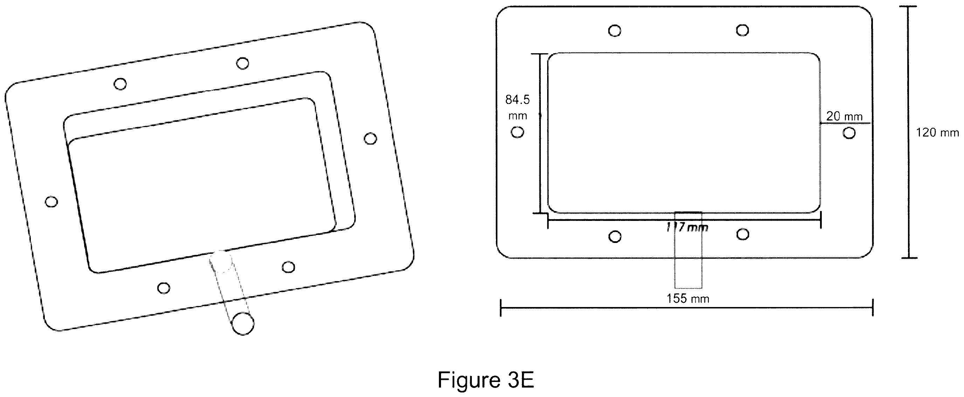

In the present, step a. of attaching and concentrating fragmented and isolated cell membranes onto the test surface can be carried out by any filtration method known from one skilled in the art. It may be for example carried out by pouring the first solution onto the test surface and leaving the solution to migrate through the test surface. For example, the first solution may be poured onto one face of the test surface until the solution has completely migrated from said surface to the opposite one and be eliminated. The surface on which the first solution is poured defined therefor the top face of the test surface. For example, the solution may be poured onto one face of the test surface and migrate under vacuum pressure. Advantageously, when vacuum is applied it allows to accelerate the migration of the solution from one face to the other. For example, step a. may be carried out using a four-window slot device as represented in Figure 3. Advantageously, the use of a four-window slot device as illustrated in Figure 3 allows a uniform deposition and concentration of the fragmented cells membranes.

According to the invention following receptor deposition, the attaching step a. can be carried out for a predetermined time; for example, this step can be carried out for at least four hours and preferably at least overnight; for example, step c) can be carried out for 4 to 12 hours.

According to the invention, the attaching step a. can be carried out at specific temperature; for example, this step can be carried out at least at 25°C and preferably at least at 37°C; for example, step a) can be carried out from 25 to 37°C.

Advantageously, when step a) is carried out at room temperature, for example from 25 to 35°C, it allows the test surface to dry.

According to the invention, the attaching step a. may be involved in the manufacture of the test zone (3a).

In the present, step b. of attaching ion-channel-linked receptor and/or a voltage gated ion-channel associated with a tagged ligand of the ion-channel receptor onto the test surface can be carried out by any filtration method known from one skilled in the art. It may be for example carried out by pouring the second solution onto the test surface and leaving the solution to migrate through the test surface. For example, the second solution may be poured onto one face of the test surface until the solution has completely migrated from said surface to the opposite one and be eliminated. The surface on which the second solution is poured defined therefore the top face of the test surface.

Advantageously, step b. of attaching an ion-channel-linked receptor and/or a voltage gated ion-channel associated with a tagged ligand of the ion-channel receptor, or a tagged ligand of the ion-channel-linked receptor and/or a voltage gated ion-channel, onto the test surface can be carried out by any filtration method with applying vacuum. Advantageously, when step b. of attaching ion-channel-linked receptor and/or a voltage gated ion-channel associated with a tagged ligand of the ion-channel receptor and/or a voltage gated ion-channel or a tagged ligand of the ion-channel-linked receptor and/or a voltage gated ion-channel onto the test surface is carried out under vacuum, the vacuum allows to drive the solution to migrate through the test surface. In the present, the vacuum may be carried out by any device known by one skilled in the art and adapted therefor. It may be for example a slot device, for example a slot device as shown in Figure 3.

According to the invention, the attaching step b. can be carried out for a predetermined time; for example, this step can be carried out for at least 4 hours and preferably at least overnight; for example, step b. can be carried out for 4 to 12 hours.

According to the invention, the attaching step b. can be carried out at a specific temperature; for example, this step can be carried out at least at 25°C and preferably at least at 37°C; for example, step b. can be carried out at a temperature from 25 to 37°C.

In other words, step b may comprise attaching and concentrating an ion-channel-linked receptor and/or a voltage gated ion-channel bound with a tagged ligand of the ion-channel-linked receptor and/or of the voltage gated ion-channel, said tagged ligand being fixed on the ion-channel-linked receptor or on the voltage gated ion-channel, which are themselves fixed on the surface defining a control zone by filtration through the test surface of a second solution comprising said ion-channel-linked receptor and/or voltage gated ion-channel bound with a tagged ligand of the ion-channel-linked receptor and/or of the voltage gated ion-channelor attaching a tagged ligand of the ion-channel-linked receptor and/or of the voltage gated ion-channel, said ligand being fixed directly on the test surface defining the test control zone or a control zone (3b') by filtration through the test surface of a second solution comprising said ligand being fixed directly on the test control zone.

According to the invention, the attaching step b. may be involve in the manufacture of the test zone (3a).

In the present, step a. and/or step b. may be carried out under vacuum. In other words, when pouring the first and /or the second solution during the step a. and/or step b., this can be carried out under vacuum which advantageously allows to accelerate the migration of the solution through the test surface and/or accelerate the drying of the test surface.

In the present, step c. of attaching a conjugate comprising an enzyme coupled to a molecule which bind to a tagged ligand of an ion-channel-linked receptor and/or a voltage-gated ion-channel or which bind to an antibody directed against the neurotoxin binding site of the ion-channel-linked receptor and/or of the voltage gated ion-channel, and/or comprising a nano gold coated molecule, a carbon-black coated molecule or a fluorescent molecule that binds to a tagged ligand of an ion-channel-linked receptor and/or a voltage gated ion-channel, may be carried out by immersion of the test surface into a third solution and/or can be carried out by any method known to one skilled in the art. For example the immersion can be carried out by totally or partially immersing the test surface into the third solution.

In other words; step c. of attaching a conjugate comprising an enzyme coupled to a molecule which bind to a tagged ligand of an ion-channel-linked receptor and/or a voltage-gated ion-channel or a conjugate which binds to an antibody directed against the neurotoxin binding site of the ion-channel-linked receptor and/or of the voltage gated ion-channel, and/or selected from the group comprising a nano gold coated molecule, a carbon-black coated molecule or a fluorescent molecule that binds to a tagged ligand of an ion-channel-linked receptor and/or a voltage gated ion-channel, may be carried out by immersion of the test surface into a third solution and/or can be carried out by any method known to one skilled in the art. For example the immersion can be carried out by totally or partially immersing the test surface into the third solution.

According to the invention, the attaching step c. can be carried out for a predetermined time; for example, the immersion step can be carried out for at least 4 hours and preferably at least 12 hours; for example, step c. can be carried out for 4 to 12 hours.

According to the invention, the attaching step c. can be carried out at a specific temperature; for example, this step can be carried out at least at 15°C and preferably at least at 37°C; for example, step c) can be carried out from 15 to 37°C.

In the present, the test surface is as defined above.

In the present the fragmented and isolated cell membrane is as defined above.

In the present, the ion-channel-linked receptor and/or a voltage gated ion-channel is as defined above.

In the present the ligand of the ion-channel-linked receptor and/or of the voltage gated ion-channel is as defined above.

In the present, the tagged ligand of the ion-channel-linked receptor and/or of the voltage gated ion-channel is as defined above.

In the present, the enzyme coupled to a molecule which binds to a tagged ligand of an ion-channel-linked receptor and/or a voltage gated ion-channel is as defined above.

In the present, the substrate of the enzyme is as defined above.

In the present the first solution comprising fragmented and isolated cell membranes may be any solution known from one skilled in the art adapted to comprise fragmented and isolated cell membranes. It may, for example a solution with a pH from 6.5 to 10, for example with a pH equal to 7.5. It may, for example, be a tris buffered saline (TBS) solution comprising 150 mM sodium chloride, 50 mM Tris-HCI or Tris, pH 7.5, a phosphate buffered saline (PBS) solution comprising 130 mM sodium chloride, 10 mM sodium phosphate, pH 7.0, or a carbonate/bicarbonate buffer solution comprising 150 mM sodium chloride, 100 mM sodium carbonate, pH 9.5. The first solution may further comprise glycine, for example from 1 to 10 mM of glycine, preferably 5 mM glycine.

According to the invention, the total protein concentration in first solution may be from 10 to 500 µg/mL, for example from 25 to 100 µg/mL. According to the invention, the ionic strength of the first solution may be from 0.1 to 0.7, preferably from 0.15.to 0.2. In other words, the ionic strength of the first solution may be from 0.1 to 0.7 mol/L, preferably from 0.15.to 0.2 mol/L.

In the present the second solution comprising ion-channel-linked receptor and/or a voltage gated ion-channel associated with a tagged ligand of the ion-channel receptor and/or a voltage gated ion-channel, or a tagged ligand of the ion-channel-linked receptor and/or a voltage gated ion-channel, may be any solution known from one skilled in the art, adapted to comprise ion-channel-linked receptor and/or a voltage gated ion-channel associated with a tagged ligand of the ion-channel receptor and/or of the voltage gated ion-channel or a tagged ligand of the ion-channel-linked receptor and/or of the voltage gated ion-channel. It may, for example be a solution with a pH from 6.5 to 9 for example with a pH equal to 7.5. According to the invention, the total protein concentration in the second solution may be from 10 to 500 µg/mL, for example from 75 to 150 µg/mL.

According to the invention, the ionic strength of the second solution may be from 0.1 to 0.7, preferably from 0.15.to 0.2. In other words, the ionic strength of the second solution may be from 0.1 to 0.7 mol/L, preferably from 0.15.to 0.2 mol/L.

In the present the third solution comprising conjugate comprising an enzyme coupled to a molecule which bind to a tagged ligand of a ion-channel-linked receptor and/or a voltage-gated ion-channel or which bind to an antibody directed against the neurotoxin binding site of the ion-channel-linked receptor and/or a voltage gated ion-channel, and/or comprising a nanogold coated molecule, a carbon-black coated molecule or a fluorescent molecule that binds to a tagged ligand of a ion-channel-linked receptor and/or a voltage gated ion-channel, may be any solution known from one in the art.

In other words, the third solution comprising conjugate comprising an enzyme coupled to a molecule which bind to a tagged ligand of a ion-channel-linked receptor and/or a voltage-gated ion-channel or which bind to an antibody directed against the neurotoxin binding site of the ion-channel-linked receptor and/or a voltage gated ion-channel, and/or a conjugate selected from the group comprising a nanogold coated molecule, a carbon-black coated molecule or a fluorescent molecule that binds to a tagged ligand of a ion-channel-linked receptor and/or a voltage gated ion-channel, may be any solution known from one in the art

It may, for example be a solution with a pH from 6.5 to 9, for example with a pH equal to 7.5.

According to the invention, the total protein concentration in the third solution may be from 10 to 500 µg/mL, for example from 50 to 100 µg/mL.

According to the invention, the ionic strength of the third solution may be from 0.1 to 0.7, preferably from 0.15 to 0.2.. In other words, the ionic strength of the third solution may be from 0.1 to 0.7 mol/L, preferably from 0.15.to 0.2 mol/L.

According to the invention, the third solution may be diluted before implementing the method of manufacturing the analysis device. For example, the third solution may be diluted from 1/50 to 1/5000.

According to the invention, the method may comprise after step a. and/or b. and/or c. an additional step d. of drying the test surface.

The drying step d. may be carried out by any drying method known from one skilled in the art. It may be for example a warming step, for example at a temperature comprises between 25 to 37°C.

In the present, step d. may be carried out by any warming method known by one skilled in the art and adapted to it. It may be for example carried out by putting the test surface into an oven at a temperature above 25°C, preferably above 30°C during at least one hour, for example for 12 hours. Step d. may be for example carried out on the lab bench at room temperature, in a desiccator.

Advantageously, the inventors have demonstrated that dehydration of the support and the membrane fragment surprisingly improve the permanent fixing of the isolated and fragmented membrane, in particular Torpedo electrocyte membranes fragments, onto glass fiber support, in particular borosilicate glass fibers. In particular, the inventors have demonstrated that the improvement of permanent fixing may be due to hydrophobic interactions while drying.

According to the invention, the method may comprise before step a. and/or b. and/or c. an additional step a' of soaking the test surface.

The soaking step a'. may be carried out by pouring the test surface into a buffer solution, for example a buffer solution at pH 7,5, for example into a Tris Buffer Solution (TBS) for example TBS (50 mM Tris-HCI, 150 mM NaCl, pH 7.5).

According to the invention, the attaching steps may be carried out onto different test surfaces each defining the different zones (1) to (3) or onto one test surfaces on which the attaching is carried out on the same surface and on different and separate place of said test surface.

When the attaching steps are carried out onto separate test surfaces each defining different zones (1) to (3), the method of the present invention may further comprise a step e. of assembling and attaching the different test surfaces onto a solid support. For example the test surface each defining the supports of zones (1) to (3), the test surface zones (1) and (2) may be associated in order to overlap at one of their ends, and the other end of zone (2) overlaps with one end of zone (3). The overlapping of the various zones (1) to (3) advantageously makes it possible, when the sample is applied and/or when the free end of zone (1) is immersed in the sample, for the sample to migrate in the various zones via capillary action.

According to the invention, the abovementioned attaching steps can be carried out onto a single test surface with various zones (1) to (3) which can be attached to a solid support, for example to laminated cards, and/or included in a container comprising an orifice at the level of the zone (3) allowing the visualization of the result and a well at the level of the zone (1) for depositing the sample. Accordingly when the attaching steps are carried out onto a singled test surface with various zones (1) to (3), the method may further comprise an additional step e. of assembling and attaching the test surface onto a solid support.

According to the invention, step e. of assembling and attaching the different test surfaces onto a solid support may be carried out onto a solid plastic support.

According to the invention, step e. may comprise attaching and assembling zone (2) on top of the solid support with one of the proximal end of the test surface comprising zone (3), attaching and assembling zone (1) for depositing a sample at the other proximal end of the solid support overlapping with one end of zone (2), and attaching and assembling the absorption zone 4.

According to the invention, the solid support may be plastic solid support, the test surface glass fiber as defined above and the absorbent zone (4) an absorbent filter paper.

An example of in-vitro device obtainable according to the present invention is illustrated on figure 2.

The device of the present invention and or obtainable by the method of the present invention can be used for detecting and or quantifying neurotoxins.

In particular the lateral flow test device of the present invention allows to rapidly and simply detect neurotoxins in a sample.

Accordingly, another object of the present invention is the use of an in-vitro device of the present invention and/or an in-vitro device obtainable by the method of the present invention for detecting and or quantifying neurotoxins in a sample as defined by claims 25 and 26.

In the present, the neurotoxins are as defined above.

In the present, the sample is as defined above.

The inventors have also demonstrated that the device of the present invention also designated lateral flow test device of the present invention may be used for detecting ligand of the ion-channel-linked receptor and/or voltage gated ion-channel present into the fragmented and isolated membrane.

Accordingly, an object of the present invention is the use of an in-vitro device of the present invention and/or an in-vitro device obtainable by the method of the present invention for detecting ligands of the ion-channel-linked receptor and/or of the voltage gated ion-channel present into the fragmented and isolated membrane.

Advantageously, when the isolated and fragmented membrane is from Torpedo fish the present device may also be used in order to purify and identify nicotinic acetylcholine receptor (nAChR) ligands. Advantageously, the strong adhesion of the Torpedo electrocyte membrane fragments on the surface makes it possible to attach and trap at least one or more ligands which interact(s) with Torpedo nAChRs. The recovery of the attached ligand(s) can be carried out, for example, after washing the device according to the invention with a washing solution, for example methanol, or by using other buffers with an ionic strength, pH and detergent concentration, or competing toxins predetermined by those skilled in the art, which can release the ligands from the Torpedo nAChR.

Another object of the present invention is a method for in-vitro detecting neurotoxins using the device of the present invention comprising the steps of:
a. depositing a sample to be tested together with a labeled toxin, in particular a labeled neurotoxin onto the depositing zone (1),
b. depositing a solution comprising the substrate of the enzyme coupled to a molecule which bind to a tagged ligand of an ion-channel-linked receptor and/or a voltage gated ion-channel or which bind to an antibody directed against the neurotoxin binding site of the ion-channel-linked receptor and/or of the voltage gated ion-channel, and/or comprising a nano gold coated molecule, a carbon-black coated molecule or a fluorescent molecule that binds to a tagged ligand of an ion-channel-linked receptor and/or a voltage gated ion-channel, and
c. analyzing the test zone and the control zone, the neurotoxin being detected when the result on the test zone is different from the result on the control zone with all technical features defined in claim 27.

According to the invention the step c) of analyzing may be carried out by any method adapted and known from one skilled in the art. It may be for example carried out by monitoring/ quantifying with the naked eye or with a lateral-flow test reader.

The method for in-vitro detecting neurotoxins using the device of the present invention comprises a further step d) of detection of neurotoxin the neurotoxin being detected when the test control zone is colored and the test zone is not.

In other words, the further step d) of detection of neurotoxin allow to conclude whether neurotoxins are present or not in the sample.

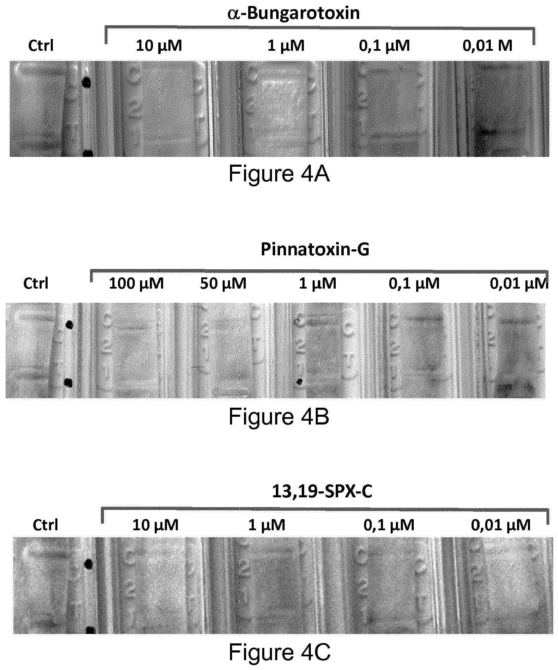

According to the invention based on the competitive binding inhibition of the labeled toxin, a lack of a colored band on the test zone denotes the presence of neurotoxin, (i.e. spirolides or anatoxin-a). There is no neurotoxin, when the test zone and the control zone are colored, and positive, i.e. there are neurotoxins into the sample, when the test zone is not colored in a same manner than the control zone, i.e. there is no coloration or the intensity of the color is less than the control zone. Figures 2B, Figures 4A, 4B and 4C represents examples of the results obtained with the method and/or device according to the present invention.

### Brief description of the Figures

Figure 1 is a schematic representation of the device for detecting neurotoxin wherein R means Torpedo nicotinic Acetylcholine Receptor, L means Biotinylated nicotinic ligand, T means toxin directed against nicotinic Acetylcholine Receptor (nAChR), S means Streptavidin-conjugate and TS means test surface. In this example, the nAChR conjugated with the biotinylated ligand was immobilized and concentrated in the control line of the GF/C membrane. Whereas, in the test line of the GF/C filter membrane, only nAChR was concentrated and immobilized. The conjugate pad was positioned between the sample pad and the GF/C filter membrane. Whenever a sample containing the biotinylated ligand and a nicotinic toxin was applied, the nicotinic toxin reacts with the binding site of the receptor displacing the biotinylated ligand towards the wicking pad. Addition of the conjugate's substrate resulted in the formation of a colored precipitate in the control zone.
Figure 2A represents a lateral schematic representation of the device. The nAChR conjugated with the biotinylated ligand was immobilized and concentrated in the control line of the GF/C membrane. Whereas, in the test line of the GF/C filter membrane, only nAChR was concentrated and immobilized. The sample pad where the sample, tagged ligands and conjugate's substrate was applied was positioned in the left extreme of the device. The conjugate pad was positioned between the sample pad and the GF/ C filter membrane. The wicking pad occupies the right extreme of the device.
Figure 2B shows a photograph of a control test without toxin and in the presence of 13-desmethyl-spirolide C. Whenever a sample containing the biotinylated ligand and a non-toxic sample was applied, the nicotinic labelled ligand reacted with the binding site of the receptor immobilized in the test line. Addition of the conjugate's substrate resulted in the formation of a colored precipitate in the test line and in the control line (Top device in the photography). Whenever a sample containing the biotinylated ligand and a nicotinic toxin was applied, the nicotinic toxin reacted with the binding site of the receptor displacing the biotinylated ligand towards the wicking pad. Addition of the conjugate's substrate resulted in the formation of a colored precipitate in the control line and a colorless or a less-colored band in the test zone (Bottom device in the photography where the effect of 2.5 µM 13-desmethyl-spirolide C is shown). The 2 cents euro coin serves as size reference of the device.
Figure 3 represents a schematic representation of a 4-wells-Slot-blot device adapted from the Hoeffer (Trademark) 48-wells-Slot-blot device for attaching and concentrating through vacuum the receptor-rich membrane fractions onto a solid support for preparing a device according to the present invention. Slots 1 and 3 were devoted to the "control zone". Slots 2 and 4 were devoted to the "test-zone". Following drying, the filter membrane (11.5 x 8.4 cm) could allow the fabrication of 40 devices according to the present invention (strips of 2.5 x 0.45 cm containing the control and tests zones).
Figure 3A represents a schematic representation of the top view of the third level of a 4-wells-Slot-blot device adapted from the Hoeffer (Trademark) 48-wells-Slot-blot device, Figure 3B represents a schematic representation of the bottom view of the third level of a 4-wells-Slot-blot device adapted from the Hoeffer (Trademark) 48-wells-Slot-blot device, Figure 3C represents a schematic representation of the top view of the second level of a 4-wells-Slot-blot device adapted from the Hoeffer (Trademark) 48-wells-Slot-blot device, Figure 3D represents a schematic representation of the bottom view of the second level of a 4-wells-Slot-blot device adapted from the Hoeffer (Trademark) 48-wells-Slot-blot device, Figure 3E represents a schematic representation of the top view of the first level of a 4-wells-Slot-blot device adapted from the Hoeffer (Trademark) 48-wells-Slot-blot device.
Figure 4 represent photographs of a dose-response detection of α-bungarotoxin, pinnatoxin-G and 13,19-didesmethyl-spirolide C by the lateral flow test of the present invention. In this case two test lines were used (there was no control line in these examples).

In Figure 4 A different doses of the snake toxin α-bungarotoxin were detected ranging from 0.01 to 10 µM. In the control test (Ctrl) where no nicotinic toxin was added both test lines were colored. At higher concentrations (10 and 1 µM) both test lines were colorless denoting 100% inhibition. At lower concentrations, both test lines are colored showing, however, a less intensity than in the control test (Ctrl).

In Figure 4B different doses of the dinoflagellate toxin pinnatoxin-G were detected ranging from 0.01 to 100 µM. In the control test (Ctrl) where no nicotinic toxin was added both test lines were colored. At higher concentrations (100 and 50 µM) both test lines were colorless denoting 100% inhibition. At lower concentrations (0.001 to 1 µM) only the upper test line was colored but to a less intensity than in the control test (Ctrl).

In Figure 4C different doses of the dinoflagellate toxin 13,19-didesmethyl-spirolide C were detected ranging from 0.01 to 10 µM. In the control test (Ctrl) where no nicotinic toxin was added both test lines were colored. At all tested concentrations, 13,19-didesmethyl-spirolide C potently inhibited the binding of the labelled ligand : 100% inhibition at 1 and 10 µM and strong inhibition at lower concentrations (0.01 to 1 µM).

### Examples

### Example 1: Method of manufacturing a device for detecting

### neurotoxins

In the example below, the products and devices used were the following:
- Alpha-bungarotoxin biotinylated (Molecular Probes, Invitrogen, USA)
- Alkaline phosphatase conjugated to streptavidin (streptavidin-AP, Promega, Madison, WI, USA)
- Alpha-bungarotoxin (Sigma-Aldrich, USA)
- TBS buffer (150 mM NaCl, 50 mM Tris-HCI, pH 7.5)
- TBS-BSA buffer (150 mM NaCl, 50 mM Tris-HCI, 0.5% BSA, pH 7.5)
- Western Blue (registered trademark) (Promega, WI, USA)
- Filter Paper Fiberglass GF/ C (Whatman, Maidstone Kent, UK)
- Filter Paper cellulose (1 mm thick, Whatman, UK)
- Headband double-sided adhesive
- Toxins standards: Anatoxin-a (Tocris), 13-desmethyl-spirolide C (NRC-IRAP, Halifax, NS, Canada), α-bungarotoxin (Sigma-Aldrich, USA), pinnatoxin-G was synthesized in Professor Armen Zakarian laboratory (University of California, Santa Barbara, California, USA, 13,19-didesmethyl-spirolide C (CIFGA, Lugo, Spain).

The isolated and fragmented membrane comprising ion-channel-linked receptor was purified from electrocyte cells of Torpedo.

The attachment and concentration of isolated and fragmented electrocyte membranes rich in nicotinic acetylcholine receptor onto the test surface was carried out by filtration. The support membrane, which was a glass filter paper GF/ C, or GF/ A or GF/ B, preferably type glass filter GF/ C (11.5 x 8.4 cm) was previously soaked into a solution of TBS (50 mM Tris-HCI, 150 mM NaCl, pH 7.5). The test surface was disposed with pliers on a slot-blot filtration device (Figure 3). The test surface was then filtered under vacuum to eliminate the excess of buffer. Immediately and still under vacuum, a volume of 3000 µL of a solution of TBS (50 mM Tris-HCI, 150 mM NaCl, pH 7.5) containing Torpedo electrocyte membranes (70 µg total protein) was applied to the long well with a 12-channel electronic pipette (250 µL in each pipette tip). The membranes of electrocyte Torpedo were retained and concentrated by the glass filter GF/ C. The test surface was then placed into an oven at 30°C overnight. Dehydration of the support and the membrane fragment surprisingly improve the permanent fixing of Torpedo membranes electrocyte fragments onto borosilicate glass fibers by hydrophobic interactions.

The attachment of electrocyte membranes rich in nicotinic acetylcholine receptor associated to a tagged ligand: the alpha-bungarotoxin biotinylated was carried out as follows:
Previously to the attachment, 3 mL of electrocyte Torpedo membranes rich in nicotinic acetylcholine receptor (35 µg/ mL) was incubated with biotinylated alpha-bungarotoxin (2 µM) overnight at 4°C. The Test surface (support membrane) type glass filter GF/ C, or GF/ A or GF/ B filter paper or, preferably type glass filter GF/ C was soaked into a solution of TBS (50 mM Tris-HCI, 150 mM NaCl, pH 7.5). The test surface was then disposed with pliers on the slot-blot filtration device as previously described.

The membrane was then filtered under vacuum. Immediately and still under vacuum, a volume of 3000 µL of a TBS solution (50 mM Tris-HCI, 150 mM NaCl, pH 7.5) containing isolated and fragmented Torpedo electrocyte membranes which has reacted with alpha-bungarotoxin biotinylated was applied to the test surface with a 12-channel electronic pipette (250 µL in each channel). The Torpedo electrocyte membranes with alpha-bungarotoxin biotinylated attached with nicotinic acetylcholine receptors were retained and concentrated by the glass filter GF/ C. The support membrane was then placed in an oven at 30 °C overnight as previously described. Dehydration of the support and the membrane fragments surprisingly improve the permanent fixing of Torpedo membranes electrocyte fragments onto borosilicate glass fibers by hydrophobic interactions.

The application of both, Torpedo electrocyte membranes and Torpedo electrocyte membranes with biotinylated alpha-bungarotoxin attached with nicotinic acetylcholine receptors, were performed at the same time as described previously. Slots 1 and 3 were devoted to the "control zone". Slots 2 and 4 were devoted to the "test-zone". Following drying, the filter membrane (11.5 x 8.4 cm) allow the fabrication of 40 devices according to the present invention (strips of 2.5 x 0.45 cm containing the control and tests zones).

The conjugate were prepared as follows. The glass filter GF/ C was also chosen to retain the alkaline streptavidin-peroxidase conjugate since the inventors surprisingly demonstrate the low ability of the glass filter GF/ C to form non-specific binding with proteins and their ability to retain large volumes and to adsorb proteins. In addition, to further reduce the irreversible adsorption of the conjugate onto the glass filter, bovine serum albumin was added to the conjugate solution. A volume of 2 mL of TBS-BSA (50 mM Tris-HCI, 150 mM NaCl, 0.5% bovine serum albumin, pH 7.5) containing alkaline phosphatase (1:500 dilution) was prepared extemporaneously.

Twelve strips of 2.1 cm long and 0.45 cm wide were immersed into the solution TBS-BSA / alkaline phosphatase. The whole was incubated for 4 h at room temperature. The strips were arranged using clamps over a piece of parafilm M (registered trademark) to minimize contact and they are dried in an oven at 30 °C overnight. The strips conjugate were stored at 4 °C until use.

Accordingly, the detection Strips as represented in Figure 1 were manufactured as follows: a double-sided adhesive tape was stuck on a plastic film of 0.1 mm. A band of 60 mm long by 0.45 cm wide was cut with a guillotine and placed on the mold after removing the protective film from the second adhesive side of the tape. A strip of glass filter GF/ C containing a test read line and a read line control of 2.5 cm long by 0.45 cm wide was deposited 1.5 cm from the edge of the sample application zone. The distance between the two sensor lines was 0.8 cm. A conjugate strip was folded in three (final size: 0.8 cm long by 0.45 cm wide) and was deposited at 0.8 cm from the sample application zone partly on the adhesive tape and the migration strip. An absorbent filter paper of 1.7 cm long by 0.45 cm wide and 1 mm thick was adhered to the sample application zone fixing the conjugate migration strip. An absorbent filter paper of 2.2 cm long by 0.45 cm wide and 1 mm thick was glued to the absorption zone and into contact with the migration strip and serves to absorb by capillarity the applied sample. Once everything was set up it was placed in a plastic housing.

In other embodiment, the detection Strips as represented in Figure 2 were manufactured as follows: the strip of glass filter GF/ C containing a "test line" and a "control line" of 25 mm long by 4.5 mm wide was mounted on a plastic band of 60 mm long, 4.5 mm width and 0.1 mm thick coated with a double-side adhesive tape. The distance between the control line and test line was 10 mm. The conjugate pad made of glass filter GF/ C of 8 mm long 4.5 mm width and 0.25 mm thick was placed at 10 mm from the test band overlapping 4 mm of the migration strip and the adhesive tape. An absorbent filter paper of 22 mm long by 4.5 mm wide and 1 mm thick was placed overlapping the conjugate pad and the adhesive band to form the so called sample application pad (Figure 1 and 2A). An absorbent filter paper of 17 mm long by 4.5 mm wide and 1 mm thick was positioned onto the other end overlapping 3 mm of the migration zone to drive by capillarity the migration of the applied solutions (Wicking pad). Once everything was set up it was placed in a plastic housing.

In another embodiment, the sample application device was modified from the 48-wells-Slot-blot device Hoeffer (Trademark). The device as conceived by Hoeffer (Trademark) was designed in three levels. The first level serves to connect the device to a vacuum apparatus or a water-trap and to collect the filtered solution. The second level as conceived by Hoeffer (Trademark), served to place the borosilicate filter membrane or whatever filter membrane was placed on it. The third level as conceived by Hoeffer (Trademark) served to apply the sample. The modification consisted in changing the 48-filter windows by four horizontal slots as illustrated in Figure 3. A borosilicate membrane previously soaked in TBS (11.5 x 8.4 cm) was layered on the top of the second level. The sample-applier was put on top of the second level containing the borosilicate membrane. The device was tightly screwed using 6-screws. The device was connected to a water-trap or to a vacuum apparatus. Vacuum was applied to eliminate the excess of water and 10 seconds later the receptor-rich membrane samples were applied. The application of both, Torpedo electrocyte membranes and Torpedo electrocyte membranes with alpha-bungarotoxin biotinylated attached with nicotinic acetylcholine receptors, were performed at the same time with a 12-channel pipette into each slot as previously described. Slots 1 and 3 were devoted to the "control zone". Slots 2 and 4 were devoted to the "test-zone". Once the samples were filtered, vacuum was cut, the device was unscrewed and the borosilicate membrane recovered and allowed to dry as described below. Once dried, the membrane was sliced to mount the lateral flow devices. The filter membrane (11.5 x 8.4 cm) allow the fabrication of 40 devices according to the present invention (strips of 2.5 x 0.45 cm containing control and tests zones). The obtained device is represented in Figure 3.

### Example 2: detection of neurotoxins with a device according to the

### present invention.

The device used was a device manufactured according to above example 1.

In the present example several experiments were carried out, a control one, an experiment without any toxin and an experiment with toxin i.e. α-bungarotoxin, spirolides, pinnatoxins, anatoxin-a.
a). Control experiment:
   - 150 µL of TBS-BSA (50 mM Tris-HCI, 150 mM NaCl, 0.5% bovine serum albumin, pH 7.5) were added in the sample wells.
   - 150 µL of substrate for alkaline phosphatase Western Blue® (Promega) were subsequently added.

   The result obtained demonstrate that the "control" line was positive which means and demonstrates that in absence of toxin, the streptavidin conjugated with alkaline phosphatase has been bound the biotin group of the complex [nicotinic acetylcholine receptor-alpha-bungarotoxin-biotin] which was immobilized on the "control" line. Hydrolysis of the Western Blue (registered trademark) substrate produces a colored precipitate in the "control" line.
b) Test experiment in absence of toxin:
   - 150 µL of TBS-BSA (50 mM Tris-HCI, 150 mM NaCl, 0.5% bovine serum albumin, pH 7.5) containing biotinylated alpha-bungarotoxin (2.4 nM) to the sample were added in the sample.
   - 150 µL of substrate for alkaline phosphatase Western Blue® (Promega) were subsequently added.

   The results obtained demonstrate the "test" and "control" lines were positive which means and demonstrates that in absence of toxin, biotinylated alpha-bungarotoxin binds to the nicotinic acetylcholine receptor immobilized on the "test" line. Thereafter the streptavidin alkaline phosphatase binded to the biotin moiety of the complex [nicotinic acetylcholine receptor - alpha-bungarotoxin-biotin] from the "test" line and that on the existing "control" line. Hydrolysis of Western Blue (registered trademark) substrate produces a colored precipitate onto both reading lines: "test" and "control" (Figure 2B, upper device).
c) Experiment in the presence of test toxin:
   - 150 µL of TBS-BSA (50 mM Tris-HCI, 150 mM NaCl, 0.5% bovine serum albumin, pH 7.5) containing biotinylated alpha-bungarotoxin (2.4 nM) and 13-desmethyl-spirolide C (2.5 µM) of the sample were added to the sample.
   - 150 µL of substrate for alkaline phosphatase Western Blue (registered trademark) (Promega) were subsequently added

The results obtained demonstrate that the "test" and "control" lines were colored but not to the same intensity or that the test line was colorless (which is an indication of high toxin content in the sample). In particular example, in the presence of 13-desmethyl-spirolide C and biotinylated alpha-bungarotoxin where both of them could bind to the nicotinic acetylcholine receptor immobilized on the "test" line, the toxin 13-desmethyl-spirolide C inhibits the binding of the tracer as a function of its concentration and its affinity thus preventing the development of a colored precipitate on test line. Consequently, if the toxin inhibition of the tracer-receptor binding was total, the Western Blue (registered trademark) substrate is hydrolyzed producing a colored precipitate only on the read line "control". Otherwise, if the inhibition toxin tracer-receptor binding was not total, the intensity of the staining on the "test" line is more or less reduced compared to the "control" line, the intensity of the coloration being inversely proportional to the toxin concentration in the sample (Figure 2B, lower device).

### Example 3: detection of neurotoxins with the device using two test lines

The device used was a device manufactured according to above example 1, with the exception that two test lines were used instead of one test line and one control line. The control line is used to control if the strip test works properly. We controlled that all the lateral flow test devices were working well and we carried out several tests without toxins that we took as control (Ctrl) that the device was working as expected (Figure 4).

In the present example several experiments were carried out using different toxins at different concentrations. Through the format two test lines, we tested the potency of the toxins at a given concentration.

In particular, different doses of the snake toxin α-bungarotoxin were tested between 0.01 to 10 µM (Figure 4A). For that purpose, 1 mL of α-bungarotoxin at a concentration of 10 µM was prepared from the commercial α-bungarotoxin (Sigma; 250 µM) using TBS-BSA buffer (150 mM NaCl, 50 mM Tris-HCI, 0.5% BSA, pH 7.5). Successively, 1 mL of 1 µM α-bungarotoxin was prepared by diluting 10-times the previous concentrated solution using TBS-BSA buffer. Serial 10-times dilutions were performed as described to obtain the 0.1 and 0.01 µM α-bungarotoxin solutions using TBS-BSA buffer.

Different doses of the dinoflagellate toxin pinnatoxin-G ranging from 0.01 to 100 µM were also tested (Figure 4B). For that purpose, 1 mL of pinnatoxin-G at a concentration of 100 µM was prepared from the synthetic pinnatoxin-G stock (A. Zakarian; 2 mM) using TBS-BSA buffer (150 mM NaCl, 50 mM Tris-HCI, 0.5% BSA, pH 7.5). Successively, 1 mL of 50 µM pinnatoxin-G was prepared by diluting 2-times the previous concentrated solution using TBS-BSA buffer. A solution of 1 µM pinnatoxin-G was obtained by diluting 50-times the previous 50 µM pinnatoxin-G solution using TBS-BSA buffer. Serial 10-times dilutions were performed as described to obtain the 0.1 and 0.01 µM pinnatoxin-G solutions using TBS-BSA buffer.

Finally, different doses of the dinoflagellate toxin 13,19-didesmethyl-spirolide C ranging from 0.01 to 10 µM were also tested. For that purpose, 1 mL of 13,19-didesmethyl-spirolide C at a concentration of 10 µM was prepared from the concentrated 13,19-didesmethyl-spirolide C stock (CIFGA; 100 µM) using TBS-BSA buffer (150 mM NaCl, 50 mM Tris-HCI, 0.5% BSA, pH 7.5). Successively, 1 mL of 1 µM 13,19-didesmethyl-spirolide C was prepared by diluting 10-times the previous concentrated solution using TBS-BSA buffer. Serial 10-times dilutions were performed as described to obtain the 0.1 and 0.01 µM 13,19-didesmethyl-spirolide C solutions using TBS-BSA buffer.

The competition experiments were carried out as disclosed in above example 2.

As demonstrated on Figure 4A, the snake toxin α-bungarotoxin inhibited the binding of the labelled tracer in both test lines at high concentrations (10 and 1 µM). At 0.1 µM, the intensity of the second test line was lower than the first test line, whereas at 1.01 µM α-bungarotoxin, both test lines show a similar intensity but lower compared to the control strip.

As shown on Figure 4B, in the case of pinnatoxin G, both test lines were colorless at 100 and 50 µM concentration, whereas at concentrations of pinnatoxin G going from 1 to 0.01 µM, only the second test line was colored with a lower intensity than was shown by the control strip.

As demonstrated on Figure 4C, the dinoflagellate toxin 13,19-didesmethyl-spirolide C inhibited the binding of the biotinylated toxin tracer at all concentration, i.e. from 0.01 to 10 µM.

Accordingly, this example clearly demonstrate that the process of the present invention and the device according to the present invention allow to detect cyclic imine toxins and advantageously allow to detect cyclic imine toxins low concentrations, for example at a concentration of 0.01 µM.

### References

1. Chen and Yang (2015) Replacing antibodies with aptamers in lateral flow immunoassay. Biosensors & bioelectronics, 71:230-242
2. Millipore 2013. Rapid lateral flow test strips: Considerations for product development. In: Lit No TB500EN00MM, Rev C: Merck Millipore.
3. Ngom B, Guo Y, Wang X, Bi D. (2010) Development and application of lateral flow test strip technology for detection of infectious agents and chemical contaminants: A review. Analytical and Bioanalytical Chemistry, 397:1113-1135.
4. Anfossi L, Baggiani C, Giovannoli C, D'Arco G, Giraudi G. 2013. Lateral-flow immunoassays for mycotoxins and phycotoxins: A review. Analytical and Bioanalytical Chemistry 405:467-480.
5. Buhrer-Sekula S, Smits HL, Gussenhoven GC, van Leeuwen J, Amador S, Fujiwara T, Klatser PR, Oskam L. (2003) Simple and fast lateral flow test for classification of leprosy patients and identification of contacts with high risk of developing leprosy. Journal of Clinical Microbiology, 41:1991-1995.
6. Marks RS, Brangel P, Papper V, Eltzov E. (2014) Electrochemical lateral flow bioassay and biosensor. In: WO2014171891 A1.
7. Directive 2010/63/EU
8. Closure of the Shellfish Farming Conference (10/15/2010). Speech by Bruno Le Maire, minister of food, agriculture and fisheries) (http://agriculture.gouv.fr/cloture-des-assises-de-la)
9. Fleming LE, Broad K, Clement A, Dewailly E, Elmir S, Knap A, Pomponi SA, Smith S, Gabriele HS, Walsh P. (2006) Oceans and human health: Emerging public health risks in the marine environment. Marine Pollution Bulletin, 53: 545-560
10. Molgó J, Girard E, Benoit E. (2007) Cyclic imines: an insight into this emerging group of bioactive marine toxins. In Phycotoxins: Chemistry and Biochemistry (Botana, L.M., ed) pp. 319-335, Blackwell Publishing Ltd, Iowa
11. Amzil Z, Sibat M, Royer F, Masson N, Abadie E. (2007) Report on the first detection of pectenotoxin-2, spirolide-A and their derivatives in French shellfish. Marine Drugs, 5:168-179
12. Bourne Y, Radic Z, Araoz R, Talley TT, Benoit E, Servent D, Taylor P, Molgó J, Marchot P. (2010) Structural determinants in phycotoxins and AChBP conferring high affinity binding and nicotinic AChR antagonism. Proceedings of the National Academy of Sciences of the United States of America, 107: 6076-6081
13. Kharrat R, Servent D, Girard E, Ouanounou G, Amar M, Marrouchi R, Benoit E, Molgó J. (2008) The marine phycotoxin gymnodimine targets muscular and neuronal nicotinic acetylcholine receptor subtypes with high affinity. Journal of Neurochemistry, 107: 952-963
14. Aráoz R, Servent D, Molgó J, lorga BI, Fruchart-Gaillard C, Benoit E, Gu Z, Stivala C, Zakarian A. (2011) Total synthesis of pinnatoxins A and G and revision of the mode of action of pinnatoxin A. Journal of the American Chemical Society, 133:10499-10511
15. Aráoz R, Botana L, Molgo J, Nghiem HO, Vilariño N. (2012) Procédé de fabrication d'un support d'analyse et utilisation pour la detection de toxines WO 2012/101378.
16. Sivonen K, Jones G. (1999) Cyanobacterial toxins. In Toxic cyanobacteria in water: a guide to their public health consequences, monitoring and management (Chorus I., ed) pp. 41-111, Bartram, J.E. & F.N. Spon, London
17. Gugger M, Lenoir S, Berger C, Ledreux A, Druart JC, Humbert JF, Guette C, Bernard C. (2005) First report in a river in France of the benthic cyanobacterium Phormidium favosum producing anatoxin-a associated with dog neurotoxicosis. Toxicon, 45: 919-928
18. Cadel-Six S, Peyraud-Thomas C, Brient L, Tandeau de Marsac N, Rippka R, Mejean A. (2007) Different genotypes of anatoxin-producing cyanobacteria coexist in the Tarn River, France. Applied and Environmental Microbiology, 73:7605-7614
19. Aráoz R, Ramos S, Pelissier F, Guérineau V, Benoit E, Vilariño N, Botana LM, Zakarian A, Molgó J. (2012). Coupling the Torpedo microplate-receptor binding assay with mass spectrometry to detect cyclic imine neurotoxins. Analytical Chemistry, 84:10445-53
20. Rubio F, Kamp L, Carpino J, Faltin E, Loftin K, Molgo J, Araoz R. (2014). Colorimetric microtiter plate receptor-binding assay for the detection of freshwater and marine neurotoxins targeting the nicotinic acetylcholine receptors. Toxicon, 91:45-56.
21. Fonfria ES, Vilariño N, Molgó J, Araoz R, Otero P, Espiña B, Louzao MC, Alvarez M, Botana LM. (2010) Detection of 13,19-didesmethyl spirolide C by fluorescence polarization using Torpedo electrocyte membranes. Analytical Biochemistry, 403:102-107
22. Vilariño N, Fonfría ES, Molgó J, Araoz R, Botana LM. (2009) Detection of gymnodimine-A and 13-desmethyl spirolide C phycotoxins by fluorescence polarization, Analytical Chemistry, 81:2708-2714
23. Aráoz R, Herdman M, Rippka R, Ledreux A, Molgó J, Changeux JP, Tandeau de Marsac N, Nghiêm HO. (2008) A non radioactive ligand-binding assay for detection of cyanobacterial anatoxins using Torpedo electrocyte membranes, Toxicon, 52:163-174
24. Aráoz R, Molgó J, Tandeau de Marsac NT. (2009) Neurotoxic cyanobacterial toxins. Toxicon, 56: 813-828

## Claims

1. An *in-vitro* lateral flow device for detecting in a sample neurotoxins or ligands of an ion-channel-linked receptor, and/or a voltage gated ion-channel comprising:
- a zone (1) for depositing a sample;
- a zone (2) comprising
∘ a conjugate comprising an enzyme coupled to a molecule which binds to a tagged ligand of an ion-channel-linked receptor, and/or a voltage gated ion-channel, or
∘ a conjugate comprising an enzyme coupled to a molecule which binds to an antibody directed against the neurotoxin binding site of the ion-channel-linked receptor and/or of the voltage gated ion-channel, and/or
∘ a conjugate comprising a nanogold coated molecule which binds to a tagged ligand of an ion-channel-linked receptor, and/or a voltage gated ion-channel, or
∘ a conjugate comprising a carbon-black coated molecule which binds to a tagged ligand of an ion-channel-linked receptor, and/or a voltage gated ion-channel, or
∘ a conjugate comprising a fluorescent molecule that binds to a tagged ligand of an ion-channel-linked receptor and/or a voltage gated ion-channel
- a visualizing zone (3) comprising
∘ a test zone (3a) comprising fragmented and isolated cell membranes comprising an ion-channel-linked receptor and/or a voltage gated ion-channel fixed on the test surface, and
∘ a control zone (3b) comprising fragmented and isolated cell membranes comprising an ion-channel-linked receptor and/or a voltage gated ion-channel bound with a tagged ligand of the ion-channel-linked receptor and/or of the voltage gated ion-channel, said ion-channel-linked receptor and/or a voltage gated ion-channel being fixed on the test surface or
∘ a control zone (3b') comprising a tagged ligand of the ion-channel-linked receptor and/or of the voltage gated ion-channel, said ligand being fixed directly on the test surface
and
- an absorption zone (4)
wherein zones (1) and (2) overlap at one of their ends, the other end of zone (2) overlaps with one end of zone (3) and absorption zone (4) overlaps with the free end of the zone (3), and
wherein the test surface is a glass fiber support or a test surface selected from the group comprising nitrocellulose membranes, mixed cellulose ester membranes, cellulose acetate membranes, hydrophilic PTFE membranes, nylon or polycarbonate membranes of high porosity.

2. The device according to claim 1, wherein the test surface is a glass fiber support

3. The device according to claim 2 , wherein the glass fiber support has pores with a diameter from 1 µm to 1,6 µm.

4. The device according to any of claim 2 to 3, wherein the glass fiber support has a thickness from 0.10 to 0.5 mm.

5. The device according to claim 1, wherein the test surface is selected from the group comprising nitrocellulose membranes, mixed cellulose ester membranes, cellulose acetate membranes, hydrophilic PTFE membranes, nylon or polycarbonate membranes of high porosity.

6. The device according to claim 5, wherein the nylon or polycarbonate membrane of high porosity comprises pores that have a diameter from 1 to 8 µm.

7. The device according to any of claims 1 to 6, wherein the ion-channel-linked receptor is selected from the group comprising ligand gated receptor channels preferably nicotinic acetylcholine receptor, or voltage-gated channels preferably voltage gated sodium channel, voltage-gated potassium channel.

8. The device according to any of claim 1 to 7, wherein the cell membrane is selected from the group comprising electrocyte cell membrane, native mammalian neuronal cells, and mammalian neuronal cells genetically modified expressing ligand gated receptor channels or voltage gated channels.

9. The device according to any of claims 1 to 8, wherein the ion-channel-linked receptor is nicotinic acetylcholine receptor and the cell membrane is Torpedo electrocyte cell membrane.

10. The device according to claim 9, wherein the surface of the test zone (3a) comprise a quantity of fragmented and isolated cell membranes from 10 to 500 µg/mL total protein.

11. The device according to claim 8 or 9, wherein the surface of the control zone (3b) comprise a quantity of fragmented and isolated Torpedo electrocyte membranes associated with a tagged ligand of the nicotinic acetylcholine receptor from 10 to 500 µg/mL.

12. A method for manufacturing an analysis device according to claim 1 - 8 comprising membrane fragments immobilized at the surface thereof, comprising the steps of:
a. Attaching and concentrating fragmented and isolated cell membranes to the test surface of the device by filtration through the test surface of a first solution comprising said fragmented and isolated cell membranes,
b. Attaching and concentrating an ion-channel-linked receptor and/or a voltage gated ion-channel bound with a tagged ligand of the ion-channel-linked receptor and/or of the voltage gated ion-channel, said ion-channel-linked receptor and/or a voltage gated ion-channel being fixed to the control test surface of the device by filtration through the test surface of a second solution comprising said ion-channel-linked receptor and/or voltage gated ion-channel bound with a tagged ligand of the ion-channel-linked receptor and/or of the voltage gated ion-channel, or
attaching a tagged ligand of the ion-channel-linked receptor and/or of the voltage gated ion-channel, said ligand being fixed directly on a test control surface.
c. attaching a conjugate comprising an enzyme coupled to a molecule which bind to a tagged ligand of an ion-channel-linked receptor and/or a voltage-gated ion-channel or a conjugate which bind to an antibody directed against the neurotoxin binding site of the ion-channel-linked receptor and/or of the voltage gated ion-channel, and/or a conjugate comprising a nanogold coated molecule, a carbon-black coated molecule or a fluorescent molecule that binds to a tagged ligand of an ion-channel-linked receptor and/or of a voltage gated ion-channel by immersion of the test surface in a third solution comprising said conjugate.
d. drying the test surface,
e. assembling and attaching the test surface onto a solid support.

13. The method according to claim 12, wherein the ion-channel-linked receptor is selected from the group comprising nicotinic acetylcholine receptor, voltage-gated sodium channel, voltage-gated potassium channel.

14. The method according to claims 12 or 13, wherein the cell membrane is selected from the group comprising electrocyte cell membrane, native mammalian neuronal cells, and mammalian neuronal cells genetically modified expressing ligand gated channel receptor channels or voltage gated channels.

15. The method according to any of claims 12 to 14, wherein the ion-channel-linked receptor is nicotinic acetylcholine receptor and the cell membrane is Torpedo electrocyte cell membrane.

16. The method according to claim 15 wherein the first solution comprise a protein concentration from 10 to 500 µg/mL of fragmented and isolated Torpedo electrocyte cell membranes.

17. The method according to claim 14 or 15 wherein the second solution comprise a protein concentration from 10 to 500 µg/mL of nicotinic acetylcholine receptor associated with a tagged ligand of the nicotinic acetylcholine receptor.

18. The method according to any of claims 15 to 17 wherein the third solution comprises a dilution from 1/50 to 1/5000 of enzyme coupled to streptavidin.

19. The method according to any of claims 12 to 18 wherein any of the filtration of step a) or b) is carried out under vacuum.

20. The method according to any of claims 12 to 19 wherein the test surface is a glass fiber support.

21. The method according to any of claims 12 to 20, wherein the glass fiber support has pores with a diameter from 1 µm to 1,6 µm.

22. The method according to any of claims 12 to 21, wherein the glass fiber support has a thickness from 0.10 to 0.50 mm.

23. The method according to any of claims 12 to 19 wherein the test surface is selected from the group comprising nitrocellulose membranes, mixed cellulose ester membranes, cellulose acetate membranes, hydrophilic PTFE membranes, nylon or polycarbonate membranes of high porosity

24. The method according to claim 23 wherein the nylon or polycarbonate membranes of high porosity comprises pores that have a diameter from 1 to 8 µm.

25. The use of an analysis device according to any one of claim 1 to 11 and/or obtainable by the method according to claim 12 to 24 for detecting and quantifying neurotoxins.

26. The use of an analysis device according to any one of claims 7 to 11 and/or obtainable by the method according to claims 12 to 24 for detecting ion-channel-linked receptor and/or a voltage gated ion-channel receptor ligands.

27. Method for in-vitro detecting neurotoxins using the device according to any of claims 1 to 11, wherein zone (2) of said device comprises a conjugate comprising an enzyme coupled to a molecule which binds to a tagged ligand of an ion-channel-linked receptor and/or of a voltage gated ion-channel or which binds to an antibody directed against the neurotoxin binding site of the ion-channel-linked receptor and/or of a voltage gated ion-channel, and wherein said method comprises the steps of:
a. depositing of a sample to be tested together with a labeled neurotoxin to be detected onto the depositing zone (1),
b. depositing a solution comprising the substrate of the enzyme coupled to a molecule which bind to a tagged ligand of an ion-channel-linked receptor and/or of a voltage gated ion-channel or which bind to an antibody directed against the neurotoxin binding site of the ion-channel-linked receptor and/or of a voltage gated ion-channel,
c. analyzing the test zone and the control zone,
d. detection of the neurotoxin, the neurotoxin being detected when the test control zone is colored and the test zone is not colored.

## Patentansprüche

1. *In-vitro* Lateralfluss-Vorrichtung zum Detektieren von Neurotoxinen oder Liganden eines ionenkanalgekoppelten Rezeptors und/oder eines spannungsgesteuerten Ionenkanals in einer Probe, enthaltend:
- eine Zone (1) zum Ablagern einer Probe;
- eine Zone (2), enthaltend
∘ ein Konjugat, enthaltend ein Enzym, das an ein Molekül gekoppelt ist, welches an einen markierten Liganden eines ionenkanalgekoppelten Rezeptors und/oder eines spannungsgesteuerten Ionenkanals bindet, oder
∘ ein Konjugat, enthaltend ein Enzym, das an ein Molekül gekoppelt ist, welches an einen Antikörper bindet, der gegen die Neurotoxinbindungsstelle des ionenkanalgekoppelten Rezeptors und/oder des spannungsgesteuerten Ionenkanals gerichtet ist, und/oder
∘ ein Konjugat, enthaltend ein Nanogold-Beschichtet-Molekül, welches an einen markierten Liganden eines ionenkanalgekoppelten Rezeptors und/oder eines spannungsgesteuerten Ionenkanals bindet, oder
∘ ein Konjugat, enthaltend ein Ruß-Beschichtet-Molekül, welches an einen markierten Liganden eines ionenkanalgekoppelten Rezeptors und/oder eines spannungsgesteuerten Ionenkanals bindet, oder
∘ ein Konjugat, enthaltend ein fluoreszierendes Molekül, das an einen markierten Liganden eines ionenkanalgekoppelten Rezeptors und/oder eines spannungsgesteuerten Ionenkanals bindet
- eine Visualisierungszone (3), enthaltend
∘ eine Prüfzone (3a), enthaltend fragmentierte und isolierte Zellmembranen, enthaltend einen ionenkanalgekoppelten Rezeptor und/oder einen spannungsgesteuerten Ionenkanal, fixiert auf der Prüfoberfläche, und
∘ eine Kontrollzone (3b), enthaltend fragmentierte und isolierte Zellmembranen, enthaltend einen ionenkanalgekoppelten Rezeptor und/oder einen spannungsgesteuerten Ionenkanal, gebunden mit einem markierten Liganden des ionenkanalgekoppelten Rezeptors und/oder des spannungsgesteuerten Ionenkanals, wobei der besagte ionenkanalgekoppelte Rezeptor und/oder ein spannungsgesteuerter Ionenkanal an der Prüfoberfläche fixiert ist oder
∘ eine Kontrollzone (3b'), enthaltend einen markierten Liganden des ionenkanalgekoppelten Rezeptors und/oder des spannungsgesteuerten Ionenkanals, wobei der besagte Ligand direkt auf der Prüfoberfläche fixiert ist
- eine Absorptionszone (4)
wobei die Zonen (1) und (2) an einem ihrer Enden überlappen, das andere Ende der Zone (2) mit einem Ende der Zone (3) überlappt und die Absorptionszone (4) mit dem freien Ende der Zone (3) überlappt, und
wobei die Prüfoberfläche ein Glasfaserträger ist oder eine Prüfoberfläche, ausgewählt aus der Gruppe enthaltend Nitrocellulose-Membranen, gemischte Cellulose-Ester-Membranen, Cellulose-Acetat-Membranen, hydrophile PTFE-Membranen, Nylon- oder Polycarbonat-Membranen mit hoher Porosität.

2. Vorrichtung gemäß Anspruch 1, wobei die Prüfoberfläche ein Glasfaserträger ist.

3. Vorrichtung gemäß Anspruch 2, wobei der Glasfaserträger Poren mit einem Durchmesser von 1 µm bis 1,6 µm hat.

4. Vorrichtung gemäß irgendeinem der Ansprüche 2 bis 3, wobei der Glasfaserträger eine Dicke von 0,10 bis 0,5 mm hat.

5. Vorrichtung gemäß Anspruch 1, wobei die Prüfoberfläche ausgewählt ist aus der Gruppe enthaltend Nitrocellulose-Membranen, gemischte Cellulose-Ester-Membranen, Cellulose-Acetat-Membranen, hydrophile PTFE-Membranen, Nylon- oder Polycarbonat-Membranen mit hoher Porosität.

6. Vorrichtung gemäß Anspruch 5, wobei die Nylon- oder Polycarbonat-Membran mit hoher Porosität Poren enthält, die einen Durchmesser von 1 bis 8 µm haben.

7. Vorrichtung gemäß irgendeinem der Ansprüche 1 bis 6, wobei der ionenkanalgekoppelte Rezeptor ausgewählt ist aus der Gruppe enthaltend, ligandengesteuerte Rezeptorkanäle, vorzugsweise nikotinischer Acetylcholinrezeptor, oder spannungsgesteuerte Kanäle, vorzugsweise spannungsgesteuerter Natriumkanal, spannungsgesteuerter Kaliumkanal.

8. Vorrichtung gemäß irgendeinem von Anspruch 1 bis 7, wobei die Zellmembran ausgewählt ist aus der Gruppe enthaltend, Elektrozyten-Zellmembran, native neuronale Säugetierzellen und neuronale Säugetierzellen die genetisch modifiziert sind und ligandengesteuerte Rezeptorkanäle oder spannungsgesteuerte Kanäle exprimieren.

9. Vorrichtung gemäß irgendeinem der Ansprüche 1 bis 8, wobei der ionenkanalgekoppelte Rezeptor ein nikotinischer Acetylcholinrezeptor ist und die Zellmembran eine Torpedo-Elektrozyten-Zellmembran ist.

10. Die Vorrichtung gemäß Anspruch 9, wobei die Oberfläche der Prüfzone (3a) fragmentierte und isolierte Zellmembranen in einer Menge von 10 bis 500 pg/mL Gesamtprotein enthält.

11. Vorrichtung gemäß Anspruch 8 oder 9, wobei die Oberfläche der Kontrollzone (3b) fragmentierte und isolierte Torpedo-Elektrozyt-Membranen in einer Menge von 10 bis 500 pg/mL enthält, die mit einem markierten Liganden des nikotinischen Acetylcholinrezeptors assoziiert sind.

12. Verfahren zur Herstellung einer Analysevorrichtung gemäß Anspruch 1 - 8, enthaltend Membranfragmente, die an ihrer Oberfläche immobilisiert sind, enthaltend die Schritte:
a. Anbringen und Konzentrieren von fragmentierten und isolierten Zellmembranen an der Prüfoberfläche der Vorrichtung durch Filtration einer ersten Lösung, die die besagten fragmentierten und isolierten Zellmembranen enthält, durch die Prüfoberfläche,
b. Anbringen und Konzentrieren eines ionenkanalgekoppelten Rezeptors und/oder eines spannungsgesteuerten Ionenkanals, der mit einem markierten Liganden des ionenkanalgekoppelten Rezeptors und/oder des spannungsgesteuerten Ionenkanals verbunden ist, wobei der besagte ionenkanalgekoppelten Rezeptor und/oder ein spannungsgesteuerter Ionenkanal an der Kontroll-PrüfOberfläche der Vorrichtung fixiert wird, durch Filtration einer zweiten Lösung, die den besagten ionenkanalgekoppelten Rezeptor und/oder spannungsgesteuerten Ionenkanal enthält, der mit einem markierten Liganden des ionenkanalgekoppelten Rezeptors und/oder des spannungsgesteuerten Ionenkanals verbunden ist, durch die Prüfoberfläche, oder
Anbringen eines markierten Liganden des ionenkanalgekoppelten Rezeptors und/oder des spannungsgesteuerten Ionenkanals, wobei der besagte Ligand direkt auf einer Prüf-Kontroll-Oberfläche fixiert wird,
c. Anbringen eines Konjugats enthaltend ein Enzym, das an ein Molekül gekoppelt ist, welches an einen markierten Liganden eines ionenkanalgekoppelten Rezeptors und/oder eines spannungsgesteuerten Ionenkanals bindet oder eines Konjugats, welches an einen Antikörper bindet, der gegen die Neurotoxinbindungsstelle des ionenkanalgekoppelten Rezeptors und/oder des spannungsgesteuerten Ionenkanals gerichtet ist, und/oder ein Konjugat, enthaltend ein Nanogold-Beschichtet-Molekül, ein Ruß-Beschichtet-Molekül oder ein fluoreszierendes Molekül, das an einen markierten Liganden eines ionenkanalgekoppelten Rezeptors und/oder eines spannungsgesteuerten Ionenkanals bindet, durch Eintauchen der Prüfoberfläche in eine dritte Lösung enthaltend das Konjugat,
d. Trocknen der Prüfoberfläche,
e. Aufbau und Befestigung der Prüfoberfläche auf einem festen Träger.

13. Verfahren gemäß Anspruch 12, wobei der ionenkanalgekoppelte Rezeptor ausgewählt ist aus der Gruppe enthaltend nikotinischer Acetylcholinrezeptor, spannungsgesteuerter Natriumkanal, spannungsgesteuerter Kaliumkanal.

14. Verfahren gemäß den Ansprüchen 12 oder 13, wobei die Zellmembran ausgewählt ist aus der Gruppe enthaltend Elektrozyten-Zellmembran, native neuronale Säugetierzellen und neuronale Säugetierzellen die genetisch modifiziert sind und ligandengesteuerte Rezeptorkanäle oder spannungsgesteuerte Kanäle exprimieren.

15. Verfahren gemäß irgendeinem der Ansprüche 12 bis 14, wobei der ionenkanalgekoppelte Rezeptor ein nikotinischer Acetylcholinrezeptor ist und die Zellmembran eine Torpedo-Elektrozyten-Zellmembran ist.

16. Verfahren gemäß Anspruch 15, wobei die erste Lösung eine Proteinkonzentration von 10 bis 500 pg/mL von fragmentierten und isolierten Torpedo-Elektrozyten-Zellmembranen enthält.

17. Verfahren gemäß Anspruch 14 oder 15, wobei die zweite Lösung eine Proteinkonzentration von 10 bis 500 pg/mL von nikotinischem Acetylcholinrezeptor enthält, der mit einem markierten Liganden des nikotinischen Acetylcholinrezeptors assoziiert ist.

18. Verfahren gemäß irgendeinem der Ansprüche 15 bis 17, wobei die dritte Lösung Enzym, das mit Streptavidin gekoppelt ist, in einer Verdünnung von 1/50 bis 1/5000 enthält.

19. Verfahren gemäß irgendeinem der Ansprüche 12 bis 18, wobei jegliche der Filtrationen von Schritt a) oder b) unter Vakuum durchgeführt wird.

20. Verfahren gemäß irgendeinem der Ansprüche 12 bis 19, wobei die Prüfoberfläche ein Glasfaserträger ist.

21. Verfahren gemäß irgendeinem der Ansprüche 12 bis 20, wobei der Glasfaserträger Poren mit einem Durchmesser von 1 µm bis 1,6 µm aufweist.

22. Verfahren gemäß irgendeinem der Ansprüche 12 bis 21, wobei der Glasfaserträger eine Dicke von 0,10 bis 0,50 mm aufweist.

23. Verfahren gemäß irgendeinem der Ansprüche 12 bis 19, wobei die Prüfoberfläche ausgewählt ist aus der Gruppe enthaltend Nitrocellulose-Membranen, gemischte Cellulose-Ester-Membranen, Cellulose-Acetat-Membranen, hydrophile PTFE-Membranen, Nylon- oder Polycarbonat-Membranen mit hoher Porosität.

24. Verfahren gemäß Anspruch 23, wobei die Nylon- oder Polycarbonat-Membran mit hoher Porosität Poren enthält mit einem Durchmesser von 1 bis 8 µm.

25. Verwendung einer Analysevorrichtung gemäß irgendeinem von Anspruch 1 bis 11 und/oder erhältlich durch das Verfahren gemäß Anspruch 12 bis 24 zur Detektion und Quantifizierung von Neurotoxinen.

26. Verwendung einer Analysevorrichtung gemäß irgendeinem der Ansprüche 7 bis 11 und/oder erhältlich durch das Verfahren gemäß den Ansprüchen 12 bis 24 zur Detektion eines ionenkanalgekoppelten Rezeptors und/oder spannungsgesteuerten Ionenkanal-Rezeptor-Liganden.

27. Verfahren zur in-vitro Detektion von Neurotoxinen unter Verwendung der Vorrichtung gemäß irgendeinem Ansprüche 1 bis 11, wobei Zone (2) der besagten Vorrichtung ein Konjugat, enthaltend ein Enzym, das an ein Molekül gekoppelt ist, welches an einen markierten Liganden eines ionenkanalgekoppelten Rezeptors und/oder eines spannungsgesteuerten Ionenkanals bindet, enthält, oder welches an einen Antikörper bindet, der gegen die Neurotoxinbindungsstelle des ionenkanalgekoppelten Rezeptors und/oder eines spannungsgesteuerten Ionenkanals gerichtet ist und wobei das besagte Verfahren die folgenden Schritte enthält:
a. Ablagern einer Probe, die geprüft werden soll, zusammen mit einem markierten Neurotoxin, das detektiert werden soll, auf die Ablagerungszone (1),
b. Ablagern einer Lösung, enthaltend das Substrat des Enzyms, das an ein Molekül gekoppelt ist, welches an einen markierten Liganden eines ionenkanalgekoppelten Rezeptors und/oder eines spannungsgesteuerten Ionenkanals bindet, oder welches an einen Antikörper bindet, der gegen die Neurotoxinbindungsstelle des ionenkanalgekoppelten Rezeptors und/oder eines spannungsgesteuerten Ionenkanals gerichtet ist,
c. Analysieren der Prüfzone und der Kontrollzone,
d. Detektion des Neurotoxins, wobei das Neurotoxin detektiert wird, wenn die Prüf-Kontroll-Zone gefärbt ist und die Prüfzone nicht gefärbt ist.

## Revendications

1. Dispositif à flux latéral *in vitro* pour détecter, dans un échantillon, des neurotoxines ou des ligands d'un récepteur ionotrope, et/ou d'un canal ionique voltage-dépendant comprenant :
- une zone (1) pour déposer un échantillon ;
- une zone (2) comprenant
∘ un conjugué comprenant une enzyme couplée à une molécule qui se lie à un ligand marqué d'un récepteur ionotrope, et/ou d'un canal ionique voltage-dépendant, ou
∘ un conjugué comprenant une enzyme couplée à une molécule qui se lie à un anticorps dirigé contre le site de liaison des neurotoxines du récepteur ionotrope, et/ou du canal ionique voltage-dépendant, et/ou
∘ un conjugué comprenant une molécule revêtue d'or nanométrique qui se lie à un ligand marqué d'un récepteur ionotrope, et/ou d'un canal ionique voltage-dépendant, ou
∘ un conjugué comprenant une molécule revêtue de noir de carbone qui se lie à un ligand marqué d'un récepteur ionotrope, et/ou d'un canal ionique voltage-dépendant, ou
∘ un conjugué comprenant une molécule fluorescente qui se lie à un ligand marqué d'un récepteur ionotrope, et/ou d'un canal ionique voltage-dépendant
- une zone de visualisation (3) comprenant
∘ une zone de test (3a) comprenant des membranes cellulaires fragmentées et isolées comprenant un récepteur ionotrope et/ou un canal ionique voltage-dépendant fixé sur la surface de test, et
∘ une zone de contrôle (3b) comprenant des membranes cellulaires fragmentées et isolées comprenant un récepteur ionotrope et/ou un canal ionique voltage-dépendant lié à un ligand marqué du récepteur ionotrope et/ou du canal ionique voltage-dépendant, ledit récepteur ionotrope et/ou canal ionique voltage-dépendant étant fixé sur la surface de test ou
∘ une zone de contrôle (3b') comprenant un ligand marqué du récepteur ionotrope et/ou du canal ionique voltage-dépendant, ledit ligand étant fixé directement sur la surface de test
et
- une zone d'absorption (4)
dans lequel les zones (1) et (2) se chevauchent à une de leurs extrémités, l'autre extrémité de la zone (2) chevauche une extrémité de la zone (3) et la zone d'absorption (4) chevauche l'extrémité libre de la zone (3), et
dans lequel la surface de test est un support en fibre de verre ou une surface de test sélectionnée dans le groupe comprenant des membranes de nitrocellulose, des membranes d'ester de cellulose mixte, des membranes d'acétate de cellulose, des membranes en PTFE hydrophiles, des membranes en nylon ou en polycarbonate ayant une porosité élevée.

2. Dispositif selon la revendication 1, dans lequel la surface de test est un support en fibre de verre.

3. Dispositif selon la revendication 2, dans lequel le support en fibre de verre comporte des pores d'un diamètre de 1 µm à 1,6 µm.

4. Dispositif selon l'une quelconque des revendications 2 à 3, dans lequel le support en fibre de verre possède une épaisseur de 0,10 à 0,5 mm.

5. Dispositif selon la revendication 1, dans lequel la surface de test est sélectionnée dans le groupe comprenant des membranes de nitrocellulose, des membranes d'ester de cellulose mixte, des membranes d'acétate de cellulose, des membranes en PTFE hydrophiles, des membranes en nylon ou en polycarbonate ayant une porosité élevée.

6. Dispositif selon la revendication 5, dans lequel la membrane en nylon ou en polycarbonate ayant une porosité élevée comprend des pores qui possèdent un diamètre de 1 à 8 µm.

7. Dispositif selon l'une quelconque des revendications 1 à 6, dans lequel le récepteur ionotrope est sélectionné dans le groupe comprenant des canaux-récepteurs ligand-dépendants, de préférence le récepteur nicotinique de l'acétylcholine, ou des canaux voltage-dépendants, de préférence un canal sodique voltage-dépendant, un canal potassique voltage-dépendant.

8. Dispositif selon l'une quelconque des revendications 1 à 7, dans lequel la membrane cellulaire est sélectionnée dans le groupe comprenant une membrane cellulaire d'électrocyte, des cellules neuronales de mammifère natives, et des cellules neuronales de mammifère génétiquement modifiées exprimant des canaux-récepteurs ligand-dépendants ou des canaux voltage-dépendants.

9. Dispositif selon l'une quelconque des revendications 1 à 8, dans lequel le récepteur ionotrope est le récepteur nicotinique de l'acétylcholine et la membrane cellulaire est une membrane cellulaire d'électrocyte de torpille (Torpédo).

10. Dispositif selon la revendication 9, dans lequel la surface de la zone de test (3a) comprend une quantité de membranes cellulaires fragmentées et isolées comprise entre 10 et 500 µg/ml de protéine totale.

11. Dispositif selon la revendication 8 ou 9, dans lequel la surface de la zone de contrôle (3b) comprend une quantité de membranes d'électrocyte de torpille (Torpédo) fragmentées et isolées associées à un ligand marqué du récepteur nicotinique de l'acétylcholine comprise entre 10 et 500 µg/ml.

12. Procédé de fabrication d'un dispositif d'analyse selon la revendication 1-8 comprenant des fragments membranaires immobilisés à la surface de celui-ci, comprenant les étapes consistant à :
a. attacher et concentrer des membranes cellulaires fragmentées et isolées à la surface de test du dispositif par filtration, à travers la surface de test, d'une première solution comprenant lesdites membranes cellulaires fragmentées et isolées,
b. attacher et concentrer un récepteur ionotrope et/ou un canal ionique voltage-dépendant lié à un ligand marqué du récepteur ionotrope et/ou du canal ionique voltage-dépendant, ledit récepteur ionotrope et/ou canal ionique voltage-dépendant étant fixé à la surface de test de contrôle du dispositif par filtration, à travers la surface de test, d'une deuxième solution comprenant ledit récepteur ionotrope et/ou canal ionique voltage-dépendant lié à un ligand marqué du récepteur ionotrope et/ou du canal ionique voltage-dépendant, ou attacher un ligand marqué du récepteur ionotrope et/ou du canal ionique voltage-dépendant, ledit ligand étant fixé directement sur une surface de contrôle de test,
c. attacher un conjugué comprenant une enzyme couplée à une molécule qui se lie à un ligand marqué d'un récepteur ionotrope et/ou d'un canal ionique voltage-dépendant ou un conjugué qui se lie à un anticorps dirigé contre le site de liaison des neurotoxines du récepteur ionotrope et/ou du canal ionique voltage-dépendant, et/ou un conjugué comprenant une molécule revêtue d'or nanométrique, une molécule revêtue de noir de carbone ou une molécule fluorescente qui se lie à un ligand marqué d'un récepteur ionotrope et/ou d'un canal ionique voltage-dépendant par l'immersion de la surface de test dans une troisième solution comprenant ledit conjugué,
d. sécher la surface de test,
e. assembler et attacher la surface de test sur un support solide.

13. Procédé selon la revendication 12, dans lequel le récepteur ionotrope est sélectionné dans le groupe comprenant le récepteur nicotinique de l'acétylcholine, un canal sodique voltage-dépendant, un canal potassique voltage-dépendant.

14. Procédé selon les revendications 12 ou 13, dans lequel la membrane cellulaire est sélectionnée dans le groupe comprenant une membrane cellulaire d'électrocyte, des cellules neuronales de mammifère natives, et des cellules neuronales de mammifère génétiquement modifiées exprimant des canaux-récepteurs ligand-dépendants ou des canaux voltage-dépendants.

15. Procédé selon l'une quelconque des revendications 12 à 14, dans lequel le récepteur ionotrope est le récepteur nicotinique de l'acétylcholine et la membrane cellulaire est une membrane cellulaire d'électrocyte de torpille (Torpédo).

16. Procédé selon la revendication 15, dans lequel la première solution comprend une concentration protéique de 10 à 500 µg/ml de membranes cellulaires d'électrocyte de torpille (Torpedo) fragmentées et isolées.

17. Procédé selon la revendication 14 ou 15, dans lequel la deuxième solution comprend une concentration protéique de 10 à 500 µg/ml de récepteur nicotinique de l'acétylcholine associé à un ligand marqué du récepteur nicotinique de l'acétylcholine.

18. Procédé selon l'une quelconque des revendications 15 à 17, dans lequel la troisième solution comprend une dilution de 1/50 à 1/5 000 d'une enzyme couplée à la streptavidine.

19. Procédé selon l'une quelconque des revendications 12 à 18, dans lequel l'une quelconque de la filtration de l'étape a) ou b) est réalisée sous vide.

20. Procédé selon l'une quelconque des revendications 12 à 19, dans lequel la surface de test est un support en fibre de verre.

21. Procédé selon l'une quelconque des revendications 12 à 20, dans lequel le support en fibre de verre comporte des pores possédant un diamètre de 1 µm à 1,6 µm.

22. Procédé selon l'une quelconque des revendications 12 à 21, dans lequel le support en fibre de verre possède une épaisseur de 0,10 à 0,50 mm.

23. Procédé selon l'une quelconque des revendications 12 à 19, dans lequel la surface de test est sélectionnée dans le groupe comprenant des membranes de nitrocellulose, des membranes d'ester de cellulose mixte, des membranes d'acétate de cellulose, des membranes en PTFE hydrophiles, des membranes en nylon ou en polycarbonate ayant une porosité élevée.

24. Procédé selon la revendication 23, dans lequel la membrane en nylon ou en polycarbonate ayant une porosité élevée comprend des pores qui possèdent un diamètre de 1 à 8 µm.

25. Utilisation d'un dispositif d'analyse selon l'une quelconque des revendications 1 à 11 et/ou pouvant être obtenu par le procédé selon la revendication 12 à 24 pour détecter et quantifier des neurotoxines.

26. Utilisation d'un dispositif d'analyse selon l'une quelconque des revendications 7 à 11 et/ou pouvant être obtenu par le procédé selon les revendications 12 à 24 pour détecter des ligands d'un récepteur ionotrope et/ou d'un récepteur à canaux ioniques voltage-dépendants.

27. Procédé de détection *in vitro* de neurotoxines utilisant le dispositif selon l'une quelconque des revendications 1 à 11, dans lequel la zone (2) dudit dispositif comprend un conjugué comprenant une enzyme couplée à une molécule qui se lie à un ligand marqué d'un récepteur ionotrope et/ou d'un canal ionique voltage-dépendant ou qui se lie à un anticorps dirigé contre le site de liaison des neurotoxines du récepteur ionotrope et/ou d'un canal ionique voltage-dépendant, et où ledit procédé comprend les étapes consistant à :
a. déposer un échantillon devant être testé avec une neurotoxine devant être détectée sur la zone de dépôt (1),
b. déposer une solution comprenant le substrat de l'enzyme couplée à une molécule qui se lie à un ligand marqué d'un récepteur ionotrope et/ou d'un canal ionique voltage-dépendant ou qui se lie à un anticorps dirigé contre le site de liaison des neurotoxines du récepteur ionotrope et/ou d'un canal ionique voltage-dépendant,
c. analyser la zone de test et la zone de contrôle,
d. détecter la neurotoxine, la neurotoxine étant détectée lorsque la zone de contrôle de test est colorée et la zone de test n'est pas colorée.
